**Europäisches Patentamt**

**European Patent Office**

**Office européen des brevets**

(19)

(11) Publication number : **0 682 114 A2**

(12) # EUROPEAN PATENT APPLICATION

(21) Application number : **95303059.0**

(22) Date of filing : **04.05.95**

(51) Int. Cl.⁶ : **C12N 15/19,** C07K 14/52, C12N 15/67, A61K 38/19, C12N 15/62, C12P 21/06, C12N 1/21, // (C12N1/21, C12R1:19)

(30) Priority : **09.05.94 US 240046**

(43) Date of publication of application : **15.11.95 Bulletin 95/46**

(84) Designated Contracting States : **AT BE CH DE DK ES FR GB GR IE IT LI LU NL PT SE**

(71) Applicant : **ELI LILLY AND COMPANY** **Lilly Corporate Center** **Indianapolis Indiana 46285 (US)**

(72) Inventor : **Belagaje, Rama** **7821 Mohawk Lane** **Indianapolis, Indiana 46260 (US)** Inventor : **Singh, Jai Pal** **13774 Hill Crest Court** **Carmel, Indiana 46032 (US)** Inventor : **Wiernicki, Todd Robert** **1060 West Southport Road** **Indianapolis, Indiana 46217 (US)**

(74) Representative : **Hudson, Christopher Mark et al** **Lilly Industries Limited** **European Patent Operations** **Erl Wood Manor** **Windlesham Surrey GU20 6PH (GB)** **Declaration under Rule 28(4) EPC (expert solution)**

(54) **Expression of recombinant MPF-4, CPF-4, PF-4 and precursor compounds.**

(57) The present invention includes methods and recombinant DNA molecules for production of human platelet factor-4 (PF-4), modified forms of PF-4 such as MPF-4 and CPF-4, and precursor polypeptides of PF-4, MPF-4 and CPF-4. The precursor polypeptides have the formula X-PF-4, X-MPF-4, or X-CPF-4 wherein X is a cleavable peptide leader sequence. The cleavable peptide leader sequence is preferably Met-Arg or Met-Arg-Met. The precursor polypeptides can be optionally cleaved to remove the cleavable peptide leader sequence to form biologically active native compounds or can be used without cleavage in methods to inhibit angiogenesis.

EP 0 682 114 A2

## Background of the Invention

Angiogenesis, the development of new capillary blood vessels, and uncontrolled endothelial cell growth can lead to pathological conditions. Conditions characterized by angiogenesis include growth of solid tumors, diabetic retinopathy, psoriasis, immune and non-immune inflammation, hemagianoma, and Karposis' sarcoma. Endothelial cell proliferation is associated with endometriosis and the formation of excessive scar tissue.

Several substances are known to inhibit endothelial and capillary cell growth *in vitro*. A family of proteins known as the chemokines have been found to modulate a variety of biological processes including angiogenesis, cell proliferation, coagulation, inflammation, and tissue repair. See Oppenheim et al., Ann. Rev. Immunol., 9:617 (1991); Taylor, Nature, 297:307 (1982); Maione et al., Science, 247:77 (1990); and Critical Reviews in Immunol., 12:17-46 (1992). One member of the chemokine family is known as Platelet Factor-4 (PF-4).

The complete primary structure of PF-4 is well known in the art (Poncz et al., Blood, 69:219 (1987)). Analogs and fragments of PF-4 are also well known and have been referred to as "Oncostatin-A" in U.S. Patents 4,645,828 and 4,737,580, herein incorporated by reference. Mutant forms of PF-4 have been disclosed, as have methods of using the mutants formed for treating angiogenic diseases. See U.S. Patents 5,086,164 and 5,112,946, herein incorporated by reference. These patents teach PF-4 modifications made in the C-terminal region resulting in analogs that no longer have heparin-binding capacity.

The 3-dimensional structure of bovine PF-4 has also been determined. See St. Charles et al., J. Biol. Chem., 264:2092 (1989). Studies have shown that the chemokine family of proteins contain a characteristic cysteine-X-cysteine (CXC) motif located in the *N*-terminal region. The CXC motif participates in producing the secondary and tertiary structure of native PF-4 via formation of intramolecular disulfide bonds with residues Cys-36 and Cys-51. Moreover, it was determined that the N-terminal residues Gln-9 to Val-19 form a large open loop and Thr-16 hydrogen bonds to Cys-51. These N-terminal structures have been shown to be important for the immunoregulatory activity of PF-4. It is also well known that the lysine rich C-terminal region of PF-4 strongly binds to heparin and related glycosaminoglycans. Rucinski et al., Thrombos. Haemostas., 63:493 (1990).

Processed forms of human Platelet Factor-4 have been identified. A processed form of human Platelet Factor-4 has been designated Modified Platelet Factor-4 (MPF-4). MPF-4 has the sequence shown in SEQ ID NO:4 and shares sequence identity with PF-4 at residues Ser-17 to Ser-70 and, thus, is presumed to be a naturally occurring cleavage product of PF-4. Another processed form of human PF-4 has been designated CPF-4. CPF-4 is a proteolytically cleaved, non-reduced form of PF-4. CPF-4 bears the same amino acid sequence of PF-4, but differs in that the peptide bond between residues 16 and 17 is absent. However, the two resulting peptides remain bonded via disulfide bridges. Both MPF-4 and CPF-4 may be isolated from the spent culture medium of the lipopolysaccharide stimulated peripheral blood leukocytes (PBL). These compounds are important inhibitors of endothelial cell proliferation and are anywhere from 10 to 100-fold more active than native PF-4. Use of angiogenic inhibitors such as MPF-4 and CPF-4 presents a potentially viable approach for the treatment of solid tumors.

Thus, there is a need to produce large amounts of MPF-4 and CPF-4 and related polypeptides. One way to efficiently produce large amounts of these polypeptides is to produce them by means of recombinant DNA technology. Thus, there is a need to develop DNA molecules having a sequence coding for MPF-4, CPF-4 or other related polypeptides.

## Summary of the Invention

The present invention includes methods and recombinant DNA sequences that provide for expression of PF-4, MPF-4, CPF-4 chain A, and precursor polypeptides. The precursor polypeptides have the formula X-PF-4, X-MPF-4, or X-CPF-4 chain A, wherein X is a cleavable peptide sequence. The cleavable peptide sequence functions to increase expression of recombinant polypeptides in a host cell. The recombinant precursor polypeptide can be optionally cleaved to form biologically active native compounds or can be used without cleavage to inhibit angiogenesis.

## Brief Description of the Figures

FIGURE 1: Synthetic DNA sequence coding for Met-MPF-4 (SEQ ID NO:1). The 5' and 3' ends contains recognition sequences for Ndel, HindIII, BamHI, and EcoRI. The recognition sequences for other restriction endonucleases are identified as well as the predicted amino acid sequence (SEQ ID NO:2).

FIGURE 2: Scheme for formation of expression vectors including the λ $P_L$ promoter and DNA sequences coding for Met-MPF-4 (pRB400A), Met-Arg-Met-MPF-4 (pRB401A), Met-Arg-Met-PF-4 (pRB402A), and Met-Arg-Met-PF-4* (pRB403A).

FIGURE 3: Scheme for formation of expression vectors including the T$_7$ promoter and DNA sequences coding for Met-MPF-4 (pRB400B), Met-Arg-Met-MPF-4 (pRB401B), Met-Arg-Met-PF-4 (pRB402B), and Met-Arg-Met-PF-4* (pRB403B).

FIGURE 4 shows inhibition of endothelial cell proliferation in a dose-responsive manner by recombinant Met-Arg-Met-MPF-4, heparin sepharose purified fraction.

FIGURE 5 is a graph showing inhibition of endothelial cell proliferation by recombinant MPF-4.

FIGURE 6 is a graph of fractions from heparin sepharose chromatography of recombinant Met-Arg-Met-MPF-4.

FIGURE 7 is a graph of separation of recombinant Met-Arg-Met-MPF-4 fractions from reverse phase chromatography.

FIGURE 8 is a graph showing separation of recombinant MPF-4 after cleavage and isolation by reverse phase HPLC.

## Detailed Description of the Invention

Human Platelet Factor-4 (PF-4) is a naturally occurring polypeptide including 70 amino acid residues and has the sequence as shown below (SEQ ID NO:3):

```
Glu-Ala-Glu-Glu-Asp-Gly-Asp-Leu-Gln-Cys-Leu-Cys-Val-
  1               5                    10

Lys-Thr-Thr-Ser-Gln-Val-Arg-Pro-Arg-His-Ile-Thr-Ser-
     15                  20                    25

Leu-Glu-Val-Ile-Lys-Ala-Gly-Pro-His-Cys-Pro-Thr-Ala-
          30                   35

Gln-Leu-Ile-Ala-Thr-Leu-Lys-Asn-Gly-Arg-Lys-Ile-Cys-
  40                  45                    50

Leu-Asp-Leu-Gln-Ala-Pro-Leu-Tyr-Lys-Lys-Ile-Ile-Lys-
          55                   60                    65

Lys-Leu-Leu-Glu-Ser
               70
```

Modified Platelet Factor-4 (MPF-4) is also a naturally occurring polypeptide believed to be a processed form of PF-4. The amino acid sequence of MPF-4 includes amino acid residues Ser 17 to Ser 70 of PF-4 (SEQ ID NO:4):

```
Ser-Gln-Val-Arg-Pro-Arg-His-Ile-Thr-Ser-Leu-Glu-Val-
  17           20                   25

Ile-Lys-Ala-Gly-Pro-His-Cys-Pro-Thr-Ala-Gln-Leu-Ile-
  30                 35                   40

Ala-Thr-Leu-Lys-Asn-Gly-Arg-Lys-Ile-Cys-Leu-Asp-Leu-
         45                  50                     55

Gln-Ala-Pro-Leu-Tyr-Lys-Lys-Ile-Ile-Lys-Lys-Leu-Leu-
             60                  65

Glu-Ser
   70
```

CPF-4 has the same amino acid sequence as PF-4, but includes two peptides linked by disulfide bonds. One of the peptides designated chain A has the following sequence (SEQ ID NO:7):

Glu-Ala-Glu-Glu-Asp-Gly-Asp-Leu-Gln-Cys-

Leu-Cys-Val-Lys-Thr-Thr

The other peptide designated chain B has the same amino acid sequence as MPF-4, as shown above. Chain A and chain B are connected by disulfide bonds. These disulfide bonds can be formed between Cys10 of chain A and Cys37 of chain B and/or Cys12 of chain A and Cys52 of chain B.

PF-4, MPF-4 and CPF-4 bind heparin and inhibit endothelial cell growth making them attractive candidates for therapeutic uses in treating angiogenic and cell proliferative diseases.

PF-4, MPF-4, CPF-4 chain A, and their precursor polypeptides can be prepared using solid phase protein synthesis. However, for large scale production of these compounds, recombinant DNA methodology is preferred.

Efficient expression of recombinant polypeptides, especially those of low molecular weight in bacteria, can sometimes prove difficult. The present invention, therefore, not only provides for DNA sequences coding for MPF-4, but also DNA sequences coding for precursor compounds. The precursor compounds have the formula X-MPF-4, X-PF-4 or X-CPF-4-A, wherein X is a cleavable amino acid or peptide sequence. The cleavable sequence functions to increase the expression of recombinant X-MPF-4, X-PF-4 or X-CPF-4-A in prokaryotic cells.

The cleavable sequence can contain any number of amino acid residues, preferably including from as small as a single amino acid up to about 25 amino acids. When the cleavable sequence has more than 8 amino acids, it is preferably selected to include a cleavage recognition sequence, a sequence to enhance expression in host cells, and to be similar in structure or conformation to the first 16 amino acids of PF-4. The cleavable amino acid or polypeptide sequence designated X contains at least one cleavage recognition sequence for a chemical or enzymatic cleavage agent. The cleavage sequence is preferably not found within the sequence of MPF-4, PF-4 or CPF-4-A and is preferably located so that the cleavable sequence can be removed to form biologically active native compounds if desired. The cleavage sequence is preferably located immediately adjacent to the sequence for MPF-4, PF-4 or CPF-4-A. The cleavage sequence is preferably located at the N-terminus of the polypeptide.

The N-terminal amino acid or amino acids of the cleavable sequence are selected to provide for an increase in the expression in a prokaryotic host cell. For expression in prokaryotic host cells, the first N-terminal amino acid selected is preferably methionine (Met). For an increase in expression of the recombinant MPF-4, PF-4 or CPF-4-A, the second amino acid is preferably arginine (Arg) or lysine (Lys). Optionally, the third N-terminal amino acid can be selected to function both to provide for an enhancement of expression and to serve as cleavage sequence. The third N-terminal amino acid is preferably methionine (Met).

The cleavable sequence may include other amino acids that are not selected to function to enhance expression in prokaryotic cells but may contain other cleavage recognition sequences. Preferably, those additional amino acids are those that mimic the structure and/or function of the first 16 N-terminal amino acids of PF-4. Modifications of all or part of the N-terminal sequence of PF-4 can be made including conservative amino acid substitutions as long as the sequence maintains a similar structure and/or function as the N-terminal amino acids of PF-4. Conservative amino acid substitutions known to those of skill in the art include substitutions of an amino acid of one class with an amino acid of the same class. For example, basic amino acids are Lys, Arg, and His and these amino acids can be substituted for one another. Hydrophobic amino acids are Leu, Ile, Val, Phe, Trp. Acidic amino acids are Gly and Asp. Polar amino acids are Ser and Thr. Amide amino acids are Gln or Asn. Within each class, substitution can be made. The modifications also include amino acid deletions and additions. The tertiary structure and/or function of bovine PF-4 has been characterized and, based on that structure, amino acid substitutions or deletions or additions can be selected that do not substantially alter the tertiary structure of the N-terminal domain of PF-4. The precursor polypeptide product formed is a derivative of PF-4 and can have similar biological activity to PF-4 and optionally may be cleaved to form PF-4.

Examples of the cleavable amino acid or peptide sequences include methionine (Met), methionine-arginine (Met-Arg), methionine-arginine-methionine (Met-Arg-Met) as well as:

```
Met-Arg-Met-Glu-Ala-Glu-Glu-Asp-Gly-Asp-
Leu-Gln-Cys-Leu-Cys-Val-Lys-Thr-Thr-Ser-
Gln-Met (SEQ ID NO:5), or


Met-Arg-Met-Glu-Ala-Glu-Glu-Asp-Gly-Asp-Leu-Gln-
Cys-Leu-Cys-Val-Lys-Thr-Met (SEQ ID NO:6).
```

Once the recombinant precursor peptides are expressed in transformed cells , the cleavable sequence can be optionally chemically or enzymatically cleaved to form biologically active recombinant native peptides in high yield. Alternatively, the precursor peptides can be used as biologically active compounds without cleavage.

One example of a precursor peptide that can be generated as described above has the formula X-MPF-4. The precursor peptide has a cleavable peptide or amino acid leader sequence that is preferably Met; Met-Arg; Met-Arg-Met;

```
Met-Arg-Met-Glu-Ala-Glu-Glu-Asp-Gly-Asp-
Leu-Gln-Cys-Leu-Cys-Val-Lys-Thr-Thr-Ser-
Gln-Met (SEQ ID NO:5); or


Met-Arg-Met-Glu-Ala-Glu-Glu-Asp-Gly-Asp-Leu-Gln-
Cys-Leu-Cys-Val-Lys-Thr-Met (SEQ ID NO:6).
```

The precursor polypeptide can be optionally cleaved to yield biologically active MPF-4.

Likewise, production of PF-4 by recombinant DNA methods can result in low yields. The present invention also provides for a DNA sequence coding for a precursor compound having the formula X-PF-4, wherein X is a cleavable sequence. The cleavable sequence functions to provide for enhanced expression of the recombinant peptide in a prokaryotic host cell. The cleavable sequence is preferably methionine-arginine-methionine (Met-Arg-Met). Once the recombinant precursor X-PF-4 polypeptides are expressed in transformed cells, the cleavage sequence can be optionally chemically and/or enzymatically cleaved to form biologically active recombinant PF-4 in high yields.

In another embodiment, a recombinant CPF-4 can be produced. CPF-4 is a compound that includes two peptide chains linked by disulfide bonds. A DNA having a sequence coding for the polypeptide of chain A (SEQ ID NO:7) can be designed and constructed as described previously. The present invention also provides for a DNA sequence coding for a precursor compound having a formula X-CPF-4-A, wherein X is a cleavable amino acid or peptide sequence and CPF-4-A is the polypeptide having the sequence of chain A (SEQ ID NO:7). The cleavable sequence functions to provide an increase in expression of the recombinant chain A of CPF-4. Optionally the CPF-4 chain A recombinant peptide can be expressed as a single peptide chain with multiple copies of the CPF-4 chain A connected by cleavage recognition sequences. Once the recombinant precursor chain A polypeptide is expressed in transformed cells, the cleavage sequence can optionally be cleaved to yield recombinant chain A polypeptide. The recombinant chain A polypeptide can be combined with recombinant or synthetic MPF-4 under conditions that favor disulfide bond formation to yield biologically active CPF-4.

The general scheme for preparing MPF-4, PF-4, CPF-4-A, and the precursor compounds utilizes those recombinant DNA methods well known to those of skill in the art. The steps include: (i) isolation of a natural DNA sequence coding for MPF-4, or PF-4, or chain A of CPF-4, or construction of a synthetic or semisynthetic sequence; (ii) optionally including modification of the DNA sequence coding for MPF-4, or PF-4, or chain A of CPF-4 to encode precursor compounds having a cleavable sequence; (iii) placing the DNA sequences into an expression vector and transforming an appropriate eukaryotic or prokaryotic host cell; and (iv) selecting for transformed cells and culturing the transformed cells under conditions that permit the expression of MPF-4 or precursor compounds, preferably in a high yield. The precursor compounds are then recovered, the cleavable sequences are optionally cleaved and the recombinant MPF-4, or PF-4, or chain A of CPF-4 can be refolded to native conformation.

The DNA sequence coding for MPF-4 or PF-4 or the chain A polypeptide of CPF-4 can be wholly synthetic or the result of modification to the larger native PF-4 coding for DNA. A DNA sequence that encodes native PF-4 is described in U.S. Patent 5,086,164 and can be used as starting material to obtain a DNA sequence coding for MPF-4, chain A polypeptide of CPF-4 or the precursor compounds X-MPF-4, X-PF-4, or X-CPF-4-A.

A synthetic DNA sequence coding for MPF-4 can be constructed by techniques well known in the art and as described in Example 1. See Brown et al., Methods in Enzymology, 68:109-151, (1979). DNA sequences that encode either MPF-4, PF-4 or precursor compounds can be designed based on the amino acid sequences herein disclosed and the published PF-4 sequence. It will also be understood by one of skill in the art that owing to the degeneracy of the genetic code, a sizeable yet definite number of DNA sequences can be constructed which encode MPF-4, PF-4, chain A polypeptide of CPF-4, or the precursor compounds. Once designed, the nucleotide sequence itself can be synthesized using conventional methodology.

The DNA sequences coding for MPF-4, PF-4, CPF-4, or precursor polypeptides can also be modified to contain recognition sequences for restriction enzymes that facilitate transfer of the DNA sequence into an expression vector. These recognition sequences are known to those of skill in the art and are preferably located at the 5' and/or 3' end of the coding sequence.

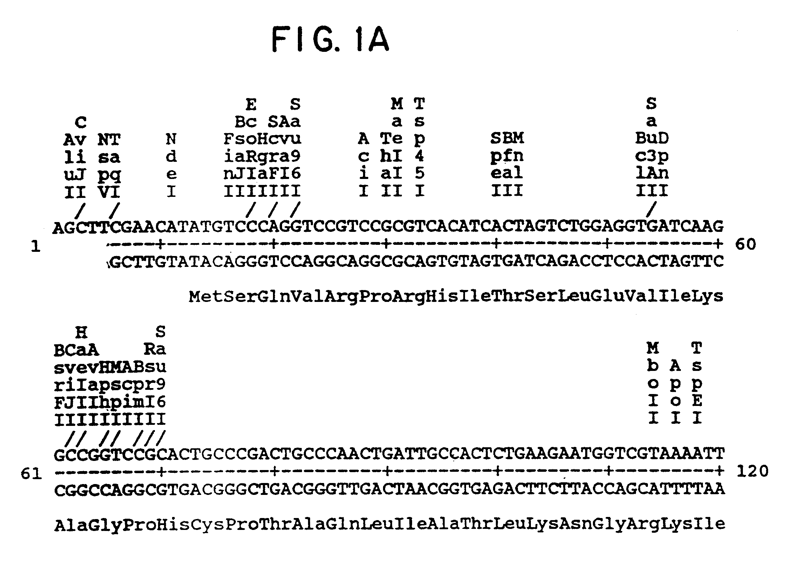

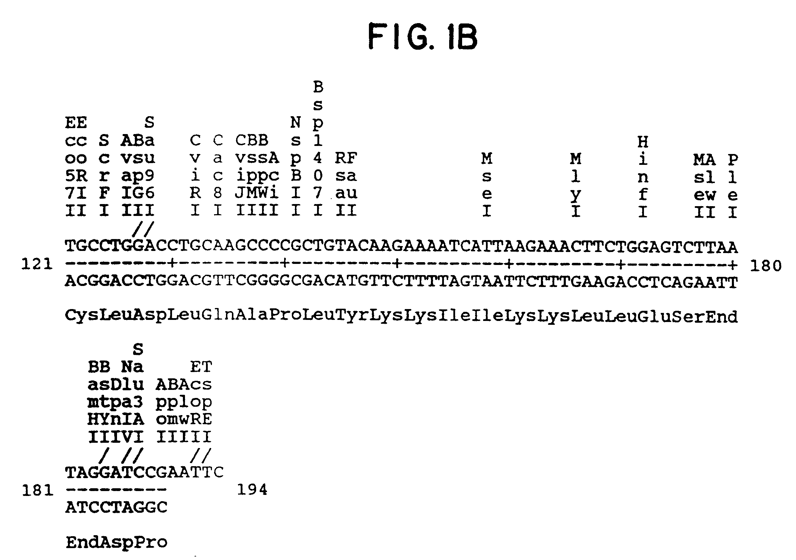

One example of a DNA sequence coding for MPF-4 is shown in Figure 1 and has SEQ ID NO:1. The DNA sequence shown in Figure 1 includes NdeI and BamHI restriction sites flanked by HindIII and EcoRI sites at the 5' and 3' ends to permit subcloning of the sequence into expression vectors. The DNA sequence shown in Figure 1 also includes a codon for methionine as the N-terminal amino acid. This DNA sequence encodes a polypeptide Met-MPF-4 having the amino acid sequence shown below (SEQ ID NO:8):

```
Met-Ser-Gln-Val-Arg-Pro-Arg-His-Ile-Thr-Ser-Leu-Glu-
  1                 5                         10

Val-Ile-Lys-Ala-Gly-Pro-His-Cys-Pro-Thr-Ala-Gln-Leu-
        15                  20                      25

Ile-Ala-Thr-Leu-Lys-Asn-Gly-Arg-Lys-Ile-Cys-Leu-Asp-
                30                  35

Leu-Gln-Ala-Pro-Leu-Tyr-Lys-Lys-Ile-Ile-Lys-Lys-Leu-
     40                  45                      50

Leu-Glu-Ser
        55
```

DNA sequences coding for the precursor molecules having a cleavable polypeptide sequence can be prepared in the same manner as described for the DNA sequence coding for PF-4 or MPF-4. One way this DNA sequence can be prepared is by modification of a natural DNA sequence coding for MPF-4 or PF-4 using techniques known to those of skill in the art such as site directed mutagenesis. Another method for preparation of the DNA sequences coding for precursor molecules is by synthetic or semisynthetic methods as described previously. The DNA sequence coding for the precursor polypeptides can be designed once a specific cleavable sequence is selected using information about the codon preference for particular host cell systems known to those of skill in the art.

A DNA sequence coding for a cleavable sequence can be designed from the amino acid sequence selected. Preferably, the cleavable leader DNA sequence encodes about 2 to 25 amino acids having at least one cleavage site cleavable by chemical or enzymatic means to provide for complete removal of the cleavable sequence. The amino acid cleavage site is one that is unique to the peptide leader sequence and is preferably not found in the native MPF-4 or PF-4 amino acid sequence. A DNA sequence encoding a cleavage recognition sequence is preferably located immediately adjacent to the DNA sequence coding for the native MPF-4 or PF-4 or chain A polypeptide of CPF-4. Preferably, the cleavage recognition sequence is upstream. For expression in prokaryotic systems, it is also preferred that the N-terminal amino acid of the cleavable leader sequence is a methionine (Met). Once the cleavable leader sequence is selected, a DNA sequence coding for that cleavage sequence can be designed using knowledge known to those of skill in the art and combined with a nucleotide sequence coding for PF-4, MPF-4, or CPF-4 chain A to form a DNA sequence coding for a precursor polypeptide. It will be understood by those of skill in the art that because of the degeneracy of the genetic code, a sizable yet definite number of DNA sequences can be constructed which encode precursor polypeptides.

In a semisynthetic method for preparing DNA sequences coding for precursor compounds having the formula X-MPF-1, the DNA sequence coding for Met-MPF-4, as shown in Figure 1, can be modified using a combination of cleaving and ligating steps known to those of skill in the art. Synthetic oligonucleotide linkers coding for a modified N-terminal amino acid sequence including a cleavable leader sequence for MPF-4 can be synthesized and inserted into the appropriate location in the DNA sequence coding for MPF-4. Several examples of oligonucleotide linkers are shown in Example 3.

Different combinations of these oligonucleotide linkers can be combined with a DNA sequence coding for MPF-4, such as shown in Figure 1, to form DNA sequences coding for precursor compounds. The oligonucleotides shown in Example 3 corresponding to MPFL-1 (SEQ ID NO:9), MPFL-2 (SEQ ID NO:10), MPFL-3 (SEQ ID NO:11), and MPFL-4 (SEQ ID NO:12) can be combined to form a DNA sequence coding for the following peptide (SEQ ID NO:13):

Met-Arg-Met-Ser-Gln-Val-Arg-Pro-Arg-His-Ile.

This DNA sequence is ligated with the DNA sequence coding for MPF-4 cut with a restriction enzyme (e.g., SpeI) that results in removal of the DNA sequence coding for the first 9 of the N-terminal amino acids of MPF-4. The resulting DNA sequence encodes a precursor polypeptide Met-Arg-Met-MPF-4 having the sequence (SEQ ID NO:14):

Met-Arg-Met-Ser-Gln-Val-Arg-Pro-Arg-His-Ile-Thr-Ser-
  1                 5                    10

Leu-Glu-Val-Ile-Lys-Ala-Gly-Pro-His-Cys-Pro-Thr-Ala-
      15              20                    25

Gln-Leu-Ile-Ala-Thr-Leu-Lys-Asn-Gly-Arg-Lys-Ile-Cys-
          30                    35

Leu-Asp-Leu-Gln-Ala-Pro-Leu-Tyr-Lys-Lys-Ile-Ile-Lys-
    40                  45                    50

Lys-Leu-Leu-Glu-Ser
        55

The Met-Arg-Met serves as a cleavable peptide sequence.

The oligonucleotides designated MPFL-3 (SEQ ID NO:11), MPFL-4 (SEQ ID NO:12), MPFL-5 (SEQ ID NO:15), and MPFL-6 (SEQ ID NO:16) having a nucleotide sequence as shown in Examples 3 and 4 can be combined to form a synthetic oligonucleotide coding for the following peptide (SEQ ID NO:17):

Met-Arg-Met-Glu-Ala-Glu-Glu-Asp-Gly-Asp-
  1               5                    10

Leu-Gln-Cys-Leu-Cys-Val-Lys-Thr-Thr-Ser-
              15                    20

Gln-Val-Arg-Pro-Arg-His-Ile
              25

This synthetic oligonucleotide can be ligated into a DNA sequence coding for MPF-4 cut with a restriction enzyme (e.g., SpeI) that results in removal of the DNA sequence coding for the first 9 N-terminal amino acids of MPF-4. The resulting DNA sequence encodes a precursor polypeptide having the amino acid sequence designated Met-Arg-Met-PF-4 (SEQ ID NO:18):

```
Met-Arg-Met-Glu-Ala-Glu-Glu-Asp-Gly-Asp-Leu-Gln-Cys-
 1                   5                        10

Leu-Cys-Val-Lys-Thr-Thr-Ser-Gln-Val-Arg-Pro-Arg-His-
    15                  20                      25

Ile-Thr-Ser-Leu-Glu-Val-Ile-Lys-Ala-Gly-Pro-His-Cys-
              30                      35

Pro-Thr-Ala-Gln-Leu-Ile-Ala-Thr-Leu-Lys-Asn-Gly-Arg-
  40                  45                  50

Lys-Ile-Cys-Leu-Asp-Leu-Gln-Ala-Pro-Leu-Tyr-Lys-Lys-
        55                  60                      65

Ile-Ile-Lys-Lys-Leu-Leu-Glu-Ser
              70
```

The Met-Arg-Met serves as the cleavable peptide sequence.

In another embodiment, oligonucleotides MPFL-5 (SEQ ID NO:15), MPFL-6 (SEQ ID NO:16), MPFL-9 (SEQ ID NO:19), and MPFL-10 (SEQ ID NO:20) having the nucleotide sequence shown in Example 4 can be combined to form a synthetic oligonucleotide linker coding for the following peptide (SEQ ID NO:21):

```
Met-Arg-Met-Glu-Ala-Glu-Glu-Asp-Gly-Asp-Leu-Gln-Cys-
 1                   5                        10

Leu-Cys-Val-Lys-Thr-Thr-Ser-Gln-Met-Ser-Gln-Val-Arg-
    15                  20                      25

Pro-Arg-His-Ile
              30
```

This synthetic oligonucleotide can be combined with a DNA sequence coding for MPF-4 that has been cut with a restriction enzyme (e.g., SpeI) removing the DNA sequence coding for the first 9 N-terminal amino acids. The resulting DNA sequence encodes a precursor polypeptide for a derivative of PF-4 designated Met-Arg-Met-PF-4*, having the amino acid sequence (SEQ ID NO:22):

```
Met-Arg-Met-Glu-Ala-Glu-Glu-Asp-Gly-Asp-Leu-Gln-Cys-
 1                   5                        10

Leu-Cys-Val-Lys-Thr-Thr-Ser-Gln-Met-Ser-Gln-Val-Arg-
    15                  20                      25

Pro-Arg-His-Ile-Thr-Ser-Leu-Glu-Val-Ile-Lys-Ala-Gly-
              30                      35

Pro-His-Cys-Pro-Thr-Ala-Gln-Leu-Ile-Ala-Thr-Leu-Lys-
  40                  45                  50

Asn-Gly-Arg-Lys-Ile-Cys-Leu-Asp-Leu-Gln-Ala-Pro-Leu-
        55                  60                      65

Tyr-Lys-Lys-Ile-Ile-Lys-Lys-Leu-Leu-Glu-Ser
              70                      75
```

8

The cleavable peptide leader sequence can either be Met-Arg-Met or

```
Met-Arg-Met-Glu-Ala-Glu-Glu-Asp-Gly-Asp-Leu-Gln-Cys-
  1                 5                      10

Leu-Cys-Val-Lys-Thr-Thr-Ser-Gln-Met     (SEQ ID NO:23).
    15                   20
```

Similarly, the oligonucleotides designated MPFL-11 (SEQ ID NO:24), MPFL-12 (SEQ ID NO:25), MPFL-3 (SEQ ID NO:11), and MPFL-4 (SEQ ID NO:12) having the nucleotide sequence as shown in Example 3 can be combined to form a synthetic oligonucleotide coding for the following peptide (SEQ ID NO:26):

```
Met-Arg-Met-Glu-Ala-Glu-Glu-Asp-Gly-Asp-Leu-Gln-
  1                 5                      10

Cys-Leu-Cys-Val-Lys-Thr-Met-Ser-Gln-Val-Arg-Pro-
        15                   20                  25

Arg-His-Ile.
```

This synthetic oligonucleotide can be combined with a DNA sequence coding for MPF-4 that has been cut with SpeI enzyme to form a DNA sequence coding for a derivative of PF-4 designated Met-Arg-Met-PF-4' having the sequence (SEQ ID NO:27):

```
Met-Arg-Met-Glu-Ala-Glu-Glu-Asp-Gly-Asp-Leu-Gln-Cys-
  1                 5                      10

Leu-Cys-Val-Lys-Thr-Met-Ser-Gln-Val-Arg-Pro-Arg-His-
        15                   20                  25

Ile-Thr-Ser-Leu-Glu-Val-Ile-Lys-Ala-Gly-Pro-His-Cys-
            30                   35

Pro-Thr-Ala-Gln-Leu-Ile-Ala-Thr-Leu-Lys-Asn-Gly-Arg-
   40                45                     50

Lys-Ile-Cys-Leu-Asp-Leu-Gln-Ala-Pro-Leu-Tyr-Lys-Lys-
        55                   60                    65

Ile-Ile-Lys-Lys-Leu-Leu-Glu-Ser
            70
```

The cleavable peptide leader sequence can be Met-Arg-Met or

```
  Met-Arg-Met-Glu-Ala-Glu-Glu-Asp-Gly-Asp-Leu-Gln-Cys-
     1                 5                      10

  Leu-Cys-Val-Lys-Thr-Met     (SEQ ID NO:28).
      15
```

The oligonucleotides designated MPFL-5 (SEQ ID NO:15) and MPFL-6 (SEQ ID NO:16) can be synthesized and combined to form a DNA sequence coding for precursor polypeptide of chain A of CPF-4 as follows:

```
5'- T ATG CGT ATG GAA GCT GAA GAA GAT GGT GAC CTG CAG TGC
3'     AC GCA TAC CTT CGA CTT CTT CTA CCA CTG GAC GTC ACG


CTG TGT GTG AAG ACC ACC TAA TAG     - 3'(SEQ ID NO:29)
GAC ACA CAC TTC TGG TGG ATT ATC CTAG - 5'(SEQ ID NO:30)
```

coding for the following polypeptide Met-Arg-Met-CPF-4 chain A (SEQ ID NO:31):

```
Met-Arg-Met-Glu-Ala-Glu-Glu-Asp-Gly-Asp-Leu-Gln-Cys-
    1               5                       10

Leu-Cys-Val-Lys-Thr-Thr.
        15
```

The cleavable leader sequence is Met-Arg-Met. Optionally, the chain A peptide can be synthesized by automated peptide synthesis and combined with recombinant MPF-4 under conditions that favor the formation of disulfide bonds.

Once the DNA sequences are obtained, they can be inserted into any one of many appropriate recombinant DNA expression vectors through the use of appropriate restriction endonucleases. See generally, Maniatis et al., Molecular Cloning: A Laboratory Manual, Cold Spring Harbor Laboratory Press, Cold Spring, NY (1989). The particular endonucleases employed will be dictated by the restriction endonuclease sites in the expression vector. The DNA sequences are inserted in frame and are operably linked to transcriptional and translational regulatory control regions which are functional in the host cell in which the protein is expressed. Appropriate transcriptional and translational control regions are known to those of skill in the art and are present in publicly available expression vectors.

A variety of expression vectors useful for transforming prokaryotic and eukaryotic cells are well known in the art. See The Promega Biological Research Products Catalogue (1992). Two expression systems that are especially preferred include an expression system containing the $\lambda P_L$ promoter and an expression system containing the $T_7$ promoter.

An expression system that includes the $\lambda P_L$ promoter is that exemplified by plasmid pCZR126S. Plasmid pCZR126S contains a $\lambda P_L$ promoter, an E. coli lipoprotein ribosome binding site, a tetracycline resistance gene and a two cistron expression system. The plasmid includes a first cistron having the following sequence (SEQ ID NO:32):

```
TCTAGAGGGT ATTAATA   ATG TAT ATT GAT TTT AAT AAG GAG GAA TAATCAT
                      Met Tyr Ile Asp Phe Asn Lys Glu Glu
```

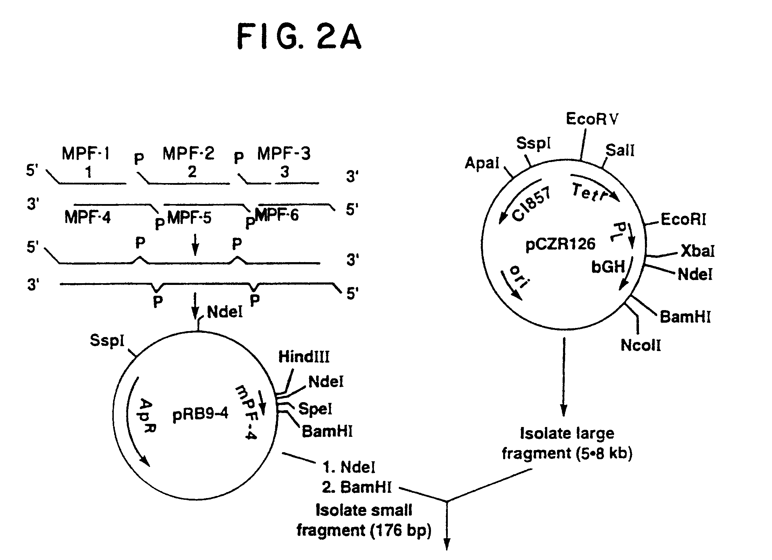

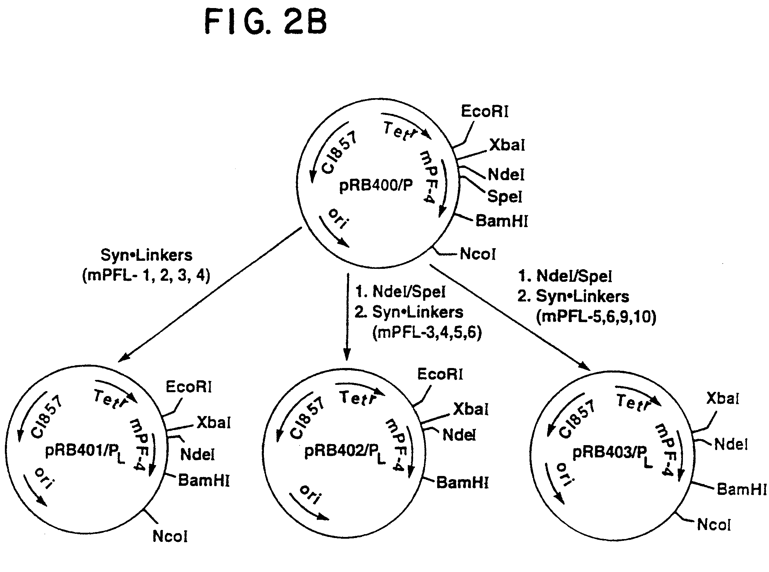

which is 5' to the MPF-4, PF-4, CPF-4 chain A, or precursor polypeptide coding sequence. Although not a requirement of the invention, the presence of the first cistron improved translation rates and, therefore, is a preferred construction. A partial restriction site and function map of pCZR126S is shown in Figure 2.

A DNA sequence coding for MPF-4, PF-4, chain A of CPF-4, or any precursor polypeptide can be inserted downstream from the $\lambda P_L$ promoters using appropriate restriction enzymes. One example of an expression vector is shown in Figure 2 and designated pRB400A. The plasmid pRB400A contains a DNA sequence coding for Met-MPF-4 downstream from the $\lambda P_L$ promoter. Plasmid pRB400A can be used to form expression vectors carrying DNA sequences coding for precursor MPF-4 or PF-4 polypeptides. An E. coli strain K12RV308 carrying the plasmid pRB400A has been deposited with Northern Regional Research Laboratories (NRRL), Peoria, Illinois and given Accession No. NRRL B-21157.

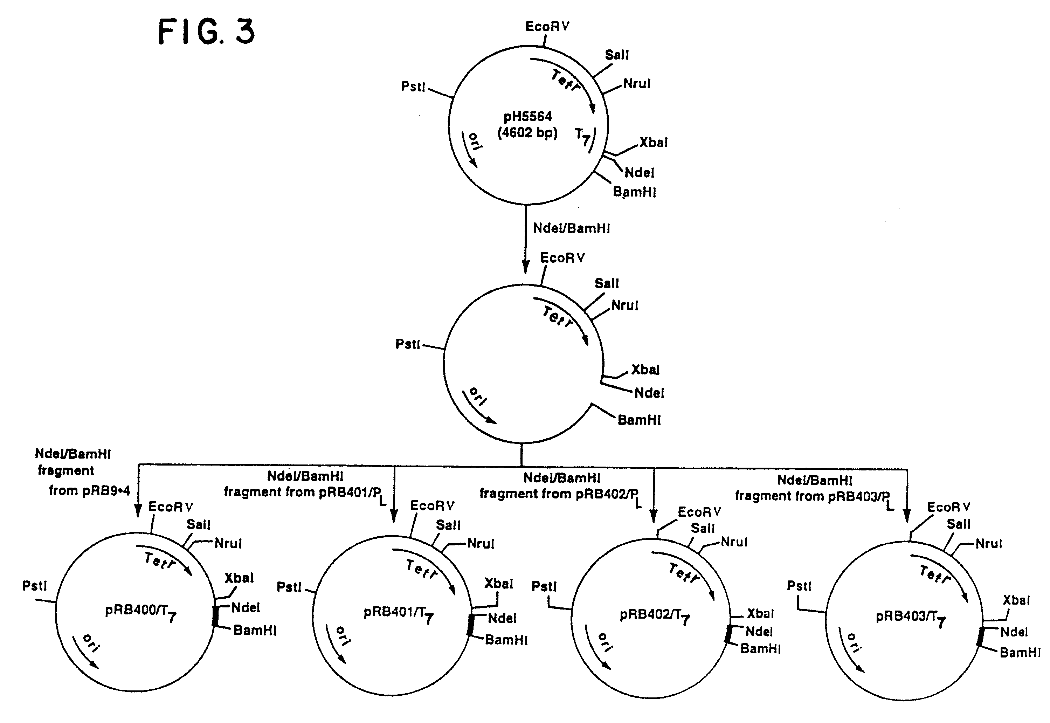

An alternative expression system that can be used to express the DNA sequences of the invention is one that has a $T_7$ promoter. One such expression system is exemplified by the plasmid pHS564 shown in Figure 3. Plasmid pHS564 is a 3.8 Kb plasmid, available from Lilly Research Laboratories, Eli Lilly and Co., Indianapolis, IN 46285. The plasmid contains the $T_7$ promoter and a tetracycline resistance gene. A partial restriction site and function map is shown in Figure 3.

A DNA sequence coding for MPF-4, PF-4, chain A of CPF-4, or precursor polypeptides can be inserted downstream from the $T_7$ promoter using appropriate restriction enzymes. An example of an expression vector is shown in Figure 3 and is designated pRB400B. Plasmid pRB400B contains the DNA sequence coding for Met-MPF-4 downstream from the $T_7$ promoter.

The present invention is not limited to the use of plasmid pCZR1226S or pHS564. Any plasmid containing or designed to contain an appropriate promoter, not just $\lambda$ $P_L$ or $T_7$, but promoters like trp, lpp and tac can also be used. Such vectors include but are not limited to pBR322. The especially preferred expression system is one that includes the $T_7$ promoter.

Once an expression vector is constructed, the vector is introduced into a suitable prokaryotic or eukaryotic host cell by transformation. Techniques for transforming host cells with recombinant DNA vectors are well known in the art and can be found in general references such as Maniatis et al., supra. Prokaryotic host cells generally produce the proteins at higher rates and are easier to culture. Suitable prokaryotic host cells include strains of E. coli such as E. coli K12RV308, E. coli BL21(DE3), E. coli BL21(DE3)(pLysS), and E. coli BL21. The preferred host cell is E. coli BL21. However, any host cell that will allow expression of the desired gene may be used. Other suitable host cells include Bacillus, Streptomyces, and yeast.

Transformed host cells that provide for a high level expression of recombinant precursor polypeptides X-MPF-4, X-PF-4, and X-CPF-4-A are E. coli BL21 cells transformed with plasmids containing DNA sequences coding for precursor polypeptides under the control of the $T_7$ promoter, or lambda $P_L$ promoter. Examples include E. coli BL21(DE3) cells transformed with pRB401B (Met-Arg-Met-MPF-4), pRB402B (Met-Arg-Met-PF-4), or pRB403D (Met-Arg-Met-PF-4*).

Recombinant proteins which are expressed or overexpressed in high level bacterial expression systems can form insoluble aggregates or inclusion bodies in the cytoplasm of transformed cells. Such protein aggregates typically must be solubilized, denatured and refolded using techniques well known in the art as described in Maniatis et al., cited supra.

In addition, the recombinant precursor polypeptides can be treated to remove the cleavable peptide leader sequence to form a recombinant polypeptide having the native MPF-4 or PF-4 or CPF-4 sequence. While cleavage of the peptide leader sequence is preferred, it may not be necessary. That is, the recombinant precursor polypeptide can have biological activity without cleavage. Several cleavage agents, chemical or enzymatic, are known in the art and can be employed to remove the peptide leader sequence. Chemical agents useful for cleaving the peptide leader sequence are cyanogen bromide, 2-(2-nitrophenyl sulfonyl)-3-bromo-3' methylindolinium (BNPS-akatole), hydroxylamine and the like. Cyanogen bromide cleaves polypeptides at the C-terminus of methionine residues. Hydroxylamine cleaves at a C-terminus of the moiety Asn-Z in which Z is Gly, Leu or Ala. BNPS-akatole cleaves at the C-terminus of a tryptophan residue.

Examples of enzymatic agents useful for cleavage are trypsin, papain, plasmin, thrombin, cathepsin C, enterokinase, and the like. Edman degradation is another cleavage method that sequentially removes single amino acids from N-terminus of a polypeptide.

For example, a recombinant precursor polypeptide Met-MPF-4 or Met-Arg-Met-MPF-4 can be cleaved with cyanogen bromide to yield recombinant MPF-4. A recombinant precursor polypeptide Met-Arg-Met-PF-4 can be cleaved with cyanogen bromide to yield recombinant PF-4. A recombinant Met-Arg-Met-PF-4* can be cleaved with cyanogen bromide to yield recombinant MPF-4 as Met-Arg-Met-PF-4* contains an internal methionine residue 22 that is cleaved and yields a peptide leader sequence and MPF-4. A recombinant Met-Arg-PF-4* can be cleaved with cathepsin C to only remove Met-Arg to yield PF-4*. A combination of cleavage reagents can be employed depending on the cleavage recognition sequence or sequences of the cleavable leader sequence and the amino sequence of MPF-4 and PF-4. Cleavage reagents are preferably selected so as not to cleave the native sequence of MPF-4 or PF-4 or CPF-4-A.

Once MPF-4, PF-4, CPF-4-A, or the precursor polypeptides have been solubilized and optionally cleaved, further purification can be utilized. Alternatively, the recombinant polypeptides can be purified and then cleaved. A great variety of protein purification techniques are well known in the art. A basic text describing protein purification techniques is Jansen and Ryder, Protein Purification, VCH Publishers, Inc., NY (1989).

One purification method includes separation of solubilized recombinant polypeptides from a cell extract by heparin-sepharose chromatography. The column is eluted with a salt buffer, preferably a linear gradient of phosphate buffered saline (PBS) and 2 M NaCl in PBS. Fractions including recombinant peptides are detected by Western blot analysis and pooled. Fractions including MPF-4, PF-4 or precursor polypeptide eluted at about .07 to 1.5 M NaCl. The pooled fractions are further separated by C-4 reverse phase high pressure liquid chromatography (HPLC). Such columns are well known in the art and may be purchased from numerous vendors including Pharmacia, BioRad, and Amicon. Upon interacting with the column, the peptide of interest is eluted using an increasing linear gradient of an organic solvent such as acetonitrile in trifluoroacetic (TFA). The preferred organic solvent is acetonitrile, and the fractions containing MPF-4 and PF-4 and the precursor polypeptides are found in the 30-50% acetonitrile portion of the gradient. Recombinant MPF-4 is isolated by C-4 reverse phase chromatography. The column is eluted with a linear gradient of 10 to 60% acetonitrile in TFA (0.05%). Recombinant MPF-4 is eluted at about 30 to 40% acetonitrile.

After each chromatographic separation, fractions are analyzed for biological activity. The recombinant precursor polypeptides have the general utility of serving as intermediates for recombinant MPF-4 or PF-4 or CPF-4. However, the recombinant precursor polypeptides can also havle angiogenesis inhibiting biological activity. The angiogenesis biological activity can be measured by determining the effect of the precursor polypeptides on endothelial cell proliferation by standard methods as described in Example 6.

Examples of recombinant precursor polypeptides include Met-Arg-Met-MPF-4 (SEQ ID NO:14), Met-MPF-4 (SEQ ID NO:8), Met-Arg-Met-PF-4 (SEQ ID NO:18), Met-Arg-Met-PF-4* (SEQ ID NO:22), Met-Arg-Met-PF-4† (SEQ ID NO:27), and Met-Arg-Met-CPF-4-A (SEQ ID NO:31). The precursor polypeptides can be optionally cleaved to yield recombinant MPF-4, PF-4, or CPF-4 chain A.

The following examples are useful for guidance and for understanding the invention. These examples are for illustrative purposes only and are not meant to limit the invention in any way.

## EXAMPLE 1

### Expression of Met-MPF-4

### A. Construction of Plasmid pRB9.4

A plasmid pRB9.4 having the DNA sequence coding for Aet-MPF-4 was prepared and selected for large scale plasmid preparation. A DNA sequence coding for Met-MPF-4 was synthetically prepared and subcloned into pUC18 (commercially available from BRL) to form pRB9.4.

### 1. Synthesis of a DNA sequence coding for Met-MPF-4

A synthetic DNA sequence coding for Met-MPF-4 was obtained by automated DNA synthesis on an ABI DNA synthesizer (model 3080 E) as follows. The synthetic DNA sequence coding for Met-MPF-4 is shown in Figure 1. The DNA sequence was selected based upon amino acid sequence information for MPF-4 including amino acids 17 to 70 of PF-4 (SEQ ID NO:4). The DNA sequence coding for MPF-4 was modified at the 5' and 3' ends by the addition of nucleotides to provide for recognition sequences for the NdeI and BamHI restriction enzymes flanked by HindIII and EcoRI recognition sequences. These recognition sequences provide for cloning of the synthetic DNA sequence into the polylinker region of the plasmid pUC18. The sequence was also modified by including a codon for methionine preceding the codon for the serine at the N terminal amino acid residue of MPF-4.

The DNA sequence coding for Met-MPF-4 was divided into six oligonucleotides of approximate length varying from 54 bases to 69 bases as shown below:

```
MPF-1 (SEQ ID NO:33):
     5' - AGC TTC GAA CAT ATG TCC CAG GTC CGT CCG CGT CAC ATC

          ACT AGT CTG GAG GTG - 3'
```

```
MPF-2  (SEQ ID NO:34):
     5' - ATC AAG GCC GGT CCG CAC TGC CCG ACT GCC CAA CTG ATT

          GCC ACT CTG AAG AAT GGT CGT AAA ATT TGC - 3'


MPF-3  (SEQ ID NO:35):
     5' - CTG GAC CTG CAA GCC CCG CTG TAC AAG AAA ATC ATT AAG

          AAA CTT CTG GAG TCT TAA TAG GAT CCG - 3'


MPF-4  (SEQ ID NO:36):
     5' - TGAT CAC CTC CAG ACT AGT GAT GTG ACG CGG ACG GAC CTG

          GGA CAT ATG TTC GA - 3'


MPF-5  (SEQ ID NO:37):
     5' - CCAG GCA AAT TTT ACG ACC ATT CTT CAG AGT GGC AAT CAG

          TTG GGC AGT CGG GCA GTG CGG ACC GGC CT - 3'


MPF-6  (SEQ ID NO:38):
     5' - AATT CGG ATC CTA TTA AGA CTC CAG AAG TTT CTT AAT GAT

          TTT CTT GTA CAG CGG GGC TTG CAG GT - 3'
```

Computer analysis using a program called GCG (available from Genetics Computer Group, Inc., Madison, WI) was used to minimize complementary sequences in positional overlapping sections. The oligonucleotides were synthesized on the ABI DNA synthesizer, gel purified and assembled into a full size product using $T_4$-DNA ligase. Briefly, 600 pmoles of each of oligonucleotides MPF-2, MPF-3, MPF-4, and MPF-5 were kinased in 50 μl volume using the standard procedure as described in Brown et al., Methods in Enzymology, cited supra., using [Gamma $P^{32}$]-ATP and polynucleotide kinase. Ligation of the oligonucleotides was conducted by mixing one nanomole of unphosphorylated MPF-1, 500 picomoles of $P^{32}$-MPF2, 500 picomoles of $P^{32}$-MPF3, 500 picomoles of $P^{32}$-MPF4, 500 picomoles of $P^{32}$-MPF5, and 1 nanomole of unphosphorylated MPF-6 in an eppendorf tube (1.5 ml) which contained 200 μl of water, 800 μM ATP, 10 mM DTT, and 50 units of $T_4$ DNA ligase. The ligation product was separated by 10% polyacrylamide gel electrophoresis. A band corresponding to 194 base pair oligonucleotide was cut out and the DNA was isolated by electroelution. The eluted DNA was passed through an Elutip-D column (Schleicher and Schuell, Keene NH 03431) and precipitated with ethanol and dried. The DNA was stored in 50 μl of 10mM Tris-HCl (pH = 7.6).

## 2. Ligation into pUC18

About 5 μg of plasmid pUC18 (commercially available from BRL) was suspended in 20 μl of 10X EcoRI buffer 100 mM NaCl, 50 mM Tris-HCl (pH 7.5), 10 mM MgCl$_2$, 1 mM dithioerythritol (DTE), 10 μg/ml bovine serum albumin (BSA)), 1 μl (10 units) of EcoRI restriction enzyme and 80 μl of water. The components were gently mixed and incubated at 37°C for 2 hours. An aliquot of this reaction mixture was checked for complete conversion of the plasmid DNA to linearized DNA on a 1% agarose gel. To the rest of the reaction mixture, 20 μl of 0.3 M sodium acetate and 1 ml of ethanol was then added. The mixture was gently mixed and kept at -70°C for 2 hours. The precipitated DNA was collected by centrifugation, washed once with 1 ml of 75% ethanol, and the pellet was dried in vacuo for about 30 minutes.

The pellet was redissolved in 80 μl of H$_2$O and 10 μl of 10X HindIII buffer (100NaCl, 10 mM Tris-HCl (pH 8.0), 10 mM MgCl$_2$, 1 mM 2-mercaptoethanol, 100 μg/ml BSA). About 1 μl (10 units) of the HindIII restriction enzyme was added and the solution was gently mixed and incubated at 37°C for 2 hours. The DNA was precipitated with 1 ml of ethanol and 20 μl of 0.3 M sodium acetate and electrophoresed on a 1% low melting agarose gel. The 2.64 Kb HindIII-EcoRI restriction fragment was sliced from the gel and the DNA was recovered by passing through an Elutip-d column using the procedure as recommended by the vendor (Schleicher and Schuell, Keene, NH 03431). After precipitation and drying, the DNA was stored in 30 μl of 10 mM Tris-HCl (pH 8.0).

About 5 μl of the HindIII/EcoRI digested plasmid pUC18 was mixed with 10 picomoles of the synthetic DNA sequence coding for Met-MPF-4 in a buffer (50 μl) containing 50 mM Tris-HCl (pH 7.6), 10 mM MgCl$_2$, 10 mM DTT, 800 μM ATP, and 3.5 units of T$_4$-DNA ligase. The reaction was incubated at 4°C overnight and the ligated plasmid DNA was transformed into E. coli K12RV308 as follows.

### 3. Preparation of Frozen, Competent E. coli K12RV308 Cells

A 250 ml portion of TY medium was inoculated with E. coli cells and the resulting culture incubated at 37°C overnight with shaking. The overnight cultures were diluted with TY medium containing 10 mM MgSO$_4$ and 10 mM MgCl$_2$ to a final volume of 250 ml, and then incubation at 37°C was continued until the OD$_{550}$ reached 0.5-0.6 absorbance units. The cells were then collected by centrifugation, washed with 125 ml of chilled 10 mM NaCl, and again collected by centrifugation. The cell pellets were resuspended in 125 ml of 30 mM CaCl$_2$ and the resulting suspension was incubated on ice for 20 to 30 minutes. The cells were then collected by centrifugation and the resulting pellets were resuspended in 12.5 ml of a cold solution of 15% glycerol in 30 mM CaCl$_2$ and 10 mM Tris-HCl (pH 8.0). The cell suspension was then aliquoted in 0.2 ml portions into prechilled tubes which were immediately placed and stored at -70°C. The cells prepared by this procedure remain viable and competent for transformation for up to one year.

### 4. Transformation

One of the tubes containing the competent E. coli cells was removed from storage at -70°C, thawed, and mixed with the recombinant plasmid DNA prepared as described above. The cell DNA mixture was incubated on ice for one hour, heat shocked at 42°C for 45 seconds, and then chilled on ice for about 2 minutes. The cell-DNA mixture was diluted into 5 ml with TY medium and incubated at 37°C for about 1 hour. 200 μl aliquots were plated on TY-agar plates containing 50 μg/ml ampicillin and the plates were placed in an incubator at 37°C until colonies appeared.

Colonies were picked from these plates and cultures grown at 37°C overnight in 3 ml of TY medium containing 100 μg/ml ampicillin. Plasmids were isolated from the cultures by the rapid alkaline extraction procedure described in Molecular Cloning, pp. 369-369. The presence of the correct Met-MPF-4 coding sequence (189 bp) was determined by HindIII-EcoRI restriction analysis of the plasmids and the desired plasmid pRB9.4 was further identified by sequence analysis. A partial restriction map of pRB9.4 is shown in Figure 2.

### B. Construction of Expression Plasmid pRB400A/λP$_L$ Expression System

Plasmid pRB400A was designed for the expression of Met-MPF-4 (SEQ ID NO:8) or other precursor polypeptides under the control of λP$_L$ promoter in E. coli K12RV308 cells and was constructed as shown in Figure 2.

Plasmid pRB9.4 was amplified in E. coli K12RV308 cells and plasmid DNA was isolated according to the method described in Maniatis et al. Molecular Cloning: A Laboratory Manual Cold Spring Harbor Laboratory, Cold Spring Harbor, NY, at pages 368-369 (1982).

About 22 μl (20 μg) of plasmid pRB9.4 DNA was added to 20 μl of 10X NdeI buffer (100 mM NaCl, 50 mM Tris-HCl (pH 7.5), 10 mM MgCl$_2$, 1 mM DTE, 100 μg/ml BSA), 5 μl (40 units) NdeI restriction enzyme and 158 μl water. After mixing, the reaction was incubated at 37°C for 2 hours. The DNA was precipitated by adding sodium acetate to a final concentration of 0.3 M, adding three volumes of ethanol, mixing and chilling to -70°C, and centrifuging. The DNA pellet was washed with 70% ethanol (1 ml); dried; and dissolved in 20 μl of 10X BamHI buffer (100 mm NaCl, 10 mM Tris-HCl (pH 8.0), 10 mM MgCl$_2$, 1 mM 2-mercaptoethanol, 100 μg/ml BSA), 2 μl (25 units) of BamHI restriction enzyme, and 160 μl water. After gentle mixing, the reaction was incubated at 37°C for 2 hours. The DNA was again precipitated with three volumes of ethanol as above and electrophoresed on a 1% low melting agarose gel. The desired DNA fragment corresponding to about 176 bp was sliced from the gel and then DNA was recovered by melting the agarose and passing through an Elutip-d column. After precipitation and drying, the purified Met-MPF-4 DNA was stored in 30 μl of 10 mM Tris-HCl (pH 7.6).

Plasmid pCZR126S contains a lambda $P_L$ promoter, an E. coli lipoprotein ribosome binding site, a tetracycline resistance gene, and a two cistron expression system as shown in Figure 2. About 15 μg of plasmid pCZR126S was suspended in 20 μl of 10X NdeI buffer. 5 μl (40 units) of NdeI restriction enzyme and 175 μl water were added, and the mixture was gently mixed and incubated at 37°C for 2 hours. After the incubation, the DNA was precipitated with three volumes of ethanol as above, dried, and then resuspended in 20 μl of 10X BamHI buffer, 2 μl (40 units) of BamHI restriction enzyme, and 178 μl water. After gentle mixing, the reaction mixture was incubated at 37°C for 2 hours. The DNA was again precipitated with three volumes of ethanol and electrophoresed on a 1% low-melting agarose gel. The 5.8 kb fragment corresponding to the NdeI-BamHI vector DNA fragment, was sliced from this gel and the DNA was recovered by the Elutip-D column procedure. After precipitation and drying, the vector DNA was stored in 35 μl of 10 mM Tris-HCl (pH 8.0).

About 3 μl of the NdeI-BamHI vector DNA fragment (5.8 kb) of pCZR126S was mixed with 5 μl of the purified met-MPF-4 gene fragment (176 bp) from above, 4 μl of 10 mM ATP, 0.5 μl of 1 M DTT, 5 μl of 10X Ligase Buffer, 26 μl of water, and 1 μl (3-5 units) of $T_4$-DNA ligase. The reaction mixture was incubated at 4°C for 16 hours. The ligated mixture was diluted with 50 μl of 10 mM Tris-HCl (pH 7.6) and 3 μl of 1 M $CaCl_2$ and a portion (50 μl) of this mixture then subsequently transformed into E. coli K12RV308 as described previously. The cells were plated on TY-agar plates supplemented with 5 μg/ml tetracycline, then incubated overnight at 32°C.

Plasmids from 3 ml cultures were isolated from tetracycline resistant colonies by the rapid alkaline extraction procedure described in Molecular Cloning:A Laboratory Manual, pp. 368-369, cited supra. The correct Met-MPF-4 gene fragment was found using polyacrylamide gel electrophoresis to analyze the NdeI-BamHI digested fragment. Those plasmids with the correct size (about 176 bp) inserts were selected for amplification and purification. The plasmid containing the Met-MPF-4 was designated pRB400A. A partial restriction site map of plasmid pRB400A shown in Figure 2.

An E. coli strain K12RV308 harboring the plasmid pRB400A is deposited with the Northern Regional Research Laboratories, Peoria, IL and given Accession No. NRRL B-21157. The plasmid pRB400A was then utilized to form an expression vector pRB400D in E. coli BL21 cells designated RB400D as follows.

About 0.5 μg of plasmid pRB400A was transformed into competent E. coli BL21 cells as described previously in Examples 1A.3 and 1B except that 2x T-Y agar plates containing 10 μg/ml tetracycline was used instead of TY agar plates. A single colony was picked up from the plate and a culture (3mL) in 2x T-Y medium containing 10 μg/ml tetracycline grown at 32°C overnight. The culture of E. coli BL21 cells containing the plasmid pRB400A was designated RB400D.

## C. Construction of pRB400B/ T7 Expression System

Plasmid pRB400B was constructed as shown in Figure 3. Plasmid pRB400B contains a DNA sequence coding for Met-MPF-4 under control of the $T_7$ promoter.

The 176 bp NdeI/BamHI restriction fragment from pRB9.4 was obtained as described previously.

Plasmid pHS564, which is a derivative of commercially available plasmid pET-3a (Novagen, Inc., Madison WI) was obtained from Mr. Dennis Smith, Lilly Research Laboratories, Eli Lilly and Co., Indianapolis, IN 46285. Plasmid pHS564 was digested with NdeI and BamHI as described for plasmid pCZR126S. A 3.76 kb fragment corresponding to the NdeI-BamHI vector DNA fragment was sliced from the gel and the DNA was recovered by the Elutip-D column procedure. After precipitation and drying, the vector was stored in 25 μl of 10 mM Tris-HCl (pH 7.6).

About 4 μl of the 3.76 Kb NdeI-BamHI vector DNA fragment of pHS564 was mixed with 5 μl of the purified 176 bp Net-MPF-4 gene fragment, 4 μl 10 mN ATP, 0.5 μl of 1 M DTT, 5 μl of 10X ligase buffer, 27 μl water, 1 μl (3.0 units) of $T_4$-DNA ligase. The reaction mixture was incubated at 4°C for 16 hours. The ligated mixture was diluted with 50 μl of 10 mM Tris-HCl (pH 7.6) and 3 μl of 1 M $CaCl_2$ and then transformed into competent E. coli BL21(DE3) cells to form pRB400B as described previously.

The transformed cells were plated on 2x T-Y agar plates supplemented with 10 μg/ml tetracycline and then incubated overnight at 37°C. Plasmids from 3 ml cultures were isolated from tetracycline resistant colonies by the rapid alkaline extraction procedure described in Molecular Cloning: A Laboratory Manual, pp. 368-369, cited supra. The correct Met-MPF-4 gene fragment was found using polyacrylamide gel electrophoresis to analyze the 176 base pair NdeI-BamHI restriction fragment. The plasmid containing the Met-MPF-4 gene was designated pRB400B. A partial restriction map is shown in Figure 3.

About 0.5 μg of plasmid pRB400B was transformed into competent E. coli BL21(DE3)(pLysS) cells. The competent cells were prepared as described in Example 1A.3 except that 2x T-Y media containing 15 μg/ml chloramphenicol was used instead of T-Y media. The transformed cells were plated on 2x T-Y agar plates supplemented with 10 μg/ml tetracycline and 15 μg/ml chloramphenicol and then incubated at 37°C. A single colony was picked and a culture (3 mL) in 2x T-Y media containing 10 μg/ml tetracycline and 15 μg/ml chloramphenicol

was grown at 37°C overnight. This culture of E. coli BL21(DE3)(pLysS) cells harboring the plasmid pRB400B was designated RB400C.

## D. Detection of Expression of Met-MPF-4

Expression of the Met-MPF-4 gene in transformed cells was examined by polyacrylamide gel electrophoresis and Western blotting. The polypeptide sequence of Met-NPF-4 is that of SEQ ID NO:8.

Transformed cells containing an expression system coding for Met-MPF-4 include E. coli K12RV308 designated as RB400A (λPL), E. coli BL21(DE3) designated as RB400B ($T_7$), BL21(DE3) (pLysS) designated RB400C ($T_7$) or E. coli BL21 designated RB400D (λPL) were grown overnight in 3 ml T-Y medium containing 5 μg/ml tetracycline (for RB400A) or 2x T-Y medium containing 10 μg/ml tetracycline (for RB400B and RB400D) or 2x T-Y medium containing 10 μg/ml tetracycline and 15 μg/ml chloramphenicol (for RB400C). About 50 μl of the overnight cultures were inoculated into 2.5 μl of appropriate T-Y medium containing tetracycline as described above and grown for 1 to 2 hours at 30°C (for λ $P_L$ expression system) or at 37°C (for $T_7$ expression system). The temperature was then shifted to 42°C for 3 hours to induce the λ $P_L$ promoter, whereas isopropyl-B-D-thiogalactoside (IPTG) was added to a final concentration of 0.4 mM to induce the $T_7$ promoter, thus resulting in expression of Met-MPF-4.

About 1 ml of the culture was pelleted and the pellet was dissolved in sample buffer (0.125 M Tris-HCl (pH 6.8)), 1 M 2-mercaptoethanol, 2% SDS, 30% glycerol, 6 M urea) to obtain $OD_{550}$ of 0.02. This solution was electrophoresed on 15% SDS polyacrylamide gel. Polypeptide bands were visualized by staining with Commassie Brilliant Blue or by Western blotting. A scanning densitometer was used to assess expression levels of the polypeptides. Western blotting was conducted using standard methods with an antibody specific for PF-4.

Low level of expression of the Met-MPF-4 in E. coli BL21(DE3) (RB400B), E. coli BL21(DE3)(pLysS) (RB400C), and E. coli BL21 (RB400D) was detected in the Western blot (data not shown).

## EXAMPLE 2

## Expression of Met-Arg-Met-MPF-4

### A. Construction of Plasmid pRB401A/λ $P_L$ Promoter Expression System

Plasmid pRB401A contains the DNA sequence coding for a MPF-4 precursor polypeptide having an N-terminal amino acid sequence Met-Arg-Met under control of the λ $P_L$ promoter. Plasmid pRB401A was constructed as follows and as shown in Figure 2.

About 20 μg of plasmid pRB400A DNA was added to 20 μl of 10X NdeI buffer (100 mM NaCl, 50 mM Tris-HCl (pH 7.5), 10 mM $MgCl_2$, 1 mM Dithioerythritol (DTE), 100 μg/ml BSA, 5 μl (40 units) NdeI restriction enzyme and 175 μl water. After mixing, the reaction was incubated at 37°C for 2 hours. The DNA was precipitated by adding sodium acetate to a final concentration of 0.3 M, adding three volumes of ethanol, mixing and chilling to -70°C, and centrifuging. The DNA pellet was washed with 70% ethanol (1 ml); dried; and dissolved in 20 μl of 10X SpeI buffer (100 mM NaCl, 50 mM Tris-HCl (pH 7.5), 10 mM $MgCl_2$, 1 mM DTE, 100 μg/ml BSA, 4 μl (40 units) of SpeI restriction enzyme, and 176 μl water. After gentle mixing, the reaction was incubated at 37°C for 2 hours. The DNA was again precipitated with three volumes of ethanol as above and electrophoresed on a 1% low melting agarose gel. The large DNA fragment (5.95 kb) was sliced from the gel and then DNA was recovered by melting the agarose and passing through an Elutip-D column. After precipitation and drying, the purified DNA was stored in 30 μl of 10 mM Tris-HCl (pH 8.0).

Oligonucleotides coding for a Met-Arg-Met cleavable peptide leader sequence for MPF-4 were synthetically prepared. Four single stranded oligonucleotides corresponding to MPFL-1, MPFL-2, MPFL-3, and MPLF-4 as shown below:

```
MPFL-1 (SEQ ID NO:9):
          5' TATGCGTATG  3'



MPFL-2 (SEQ ID NO:10):
          5'- GGGACATACGCA - 3'



MPFL-3 (SEQ ID NO:11):
          5'- TCC CAG GTC CGT CCG CGT CAC ATC A - 3'



MPFL-4 (SEQ ID NO:12):
          5'- CTAGT GAT GTG ACG CGG ACG GACCT - 3'
```

were synthesized on a 380B DNA synthesizer and purified by polyacrylamide gel electrophoresis. About 200 picomoles of the oligonucleotides 1 and 2, and 3 and 4 were phosphorylated, annealed and ligated according to the teachings of Brown et al., Methods in Enzymology cited supra., and as previously described to form the following DNA sequence (5'-3' =SEQ ID NO:39; 3'-5' = SEQ ID NO:40):

```
5' TATG CGT ATG TCC CAG GTC CGT CCG CGT CAC ATC A        3'
3'    AC GCA TAC AGG GTC CAG GCA GGC GCA GTG TAG TGATC   5'
```

coding for the first 11 N-terminal amino acids of Met-Arg-Met-MPF-4 (SEQ ID NO:14).

About 20 pmoles of this linker were mixed with 3 µl of NdeI-SpeI digested pRB400A in a buffer containing 50 mM Tris-HCl (pH 7.6), 10 mM MgCl$_2$, 10 mM DTT, 800 µm ATP and 3 units T$_4$-DNA ligase. THe reaction was incubated at 4°C overnight and then transferred into E. coli K12RV308 as described previously. The desired transformant was identified by sequence analysis of its plasmid DNA designated pRB401A. A partial restriction map of pRB401A is shown in Figure 2. An E. coli strain K12RV308 harboring the plasmid pRB401A was deposited on _____ (date) with NRRL and given Accession No. NRRLB 21158.

About 0.5 µg of plasmid pRB401A was also transformed into E. coli BL21 cells to form RB401D.

Expression in transformed E. coli K12RV308 and BL21 cells was induced as described previously. Briefly, 50 µl of overnight tetracycline resistant cultures were inoculated into 2.5 µl TY medium containing 5 µg/ml tetracycline or 2x T-Y medium containing 10 µg/ml tetracycline and grown for 1 to 2 hours at 30°C. The temperature was shifted to 42°C for 3 hours. Raising the temperature to 42°C induced the λ P$_L$ promoter, thus resulting in expression of Met-Arg-Met-MPF-4.

### B. Construction of pRB401B/T7 Expression System

Plasmid pRB401B contains the DNA sequence coding for a MPF-4 polypeptide having an N terminal amino acid sequence Met-Arg-Met under control of the T$_7$ promoter. Plasmid pRB401B was constructed as follows and as shown in Figure 3.

Plasmid pHS564 was digested with NdeI and BamHI as described in Example I. A 3.72 kb fragment corresponding to the NdeI-BamHI DNA fragment was obtained and stored in 25 µl 10 mM Tris-HCl (pH 7.6).

Plasmid pRB401A was digested with NdeI and BamHI to obtain the DNA fragment coding for the Met-Arg-Met-MPF-4 as follows. About 20 µg plasmid pRB401A in 10X NdeI buffer was mixed with 5 µl (40 units) NdeI at 37°C for 2 hours. The DNA was precipitated, washed with 70% ethanol, dried and dissolved in 20 µl 10X BamHI buffer. The DNA was dissolved and incubated with 2.5 µl (25 units) BamHI at 37°C for 2 hours. The DNA was again precipitated and electrophoresed on a 1% low melting agarose gel. The 178 bp DNA fragment corresponding to the DNA sequence coding for Met-Arg-Met-MPF-4 was sliced from the gel and recovered on an Elutip-D column.

The NdeI-BamHI fragment from plasmid pRB401A was ligated into pHS564 as described in Example 1 to form pRB401B. The sequence of plasmid pRB401B was confirmed by DNA sequence analysis. A partial restriction map of pRB401B is shown in Figure 3.

About 0.5 µg of plasmid pRB401B was transformed into competent E. coli BL21(DE3)(pLysS) cells to form RB401C. Expression of the Met-Arg-Met-MPF-4 gene was induced by culturing the transformed cells in the presence of IPTG (0.4 mM) for 4 hours. The Met-Arg-Met-MPF-4 polypeptide was detected in cell lysates by 15% SDS-polyacrylamide gel electrophoresis and staining with Commassie Brilliant Blue or by Western blotting. The polypeptide sequence of Met-Arg-Met-MPF-4 is shown in SEQ ID NO:14.

## C. Detection of Expression of Met-Arg-Met-MPF-4

Expression of the Met-Arg-Met-MPF-4 gene was detected using polyacrylamide gel electrophoresis as described previously. The expression level (% of total protein) was quantitated using scanning densitometry. The results are shown in Table I.

### TABLE I

| Plasmid | Strain | Expression Level (% of total protein) |
|---|---|---|
| pRB401A | RV 308 (RB401A) | 6% |
| pRB401B | BL21(DE3) (RB401B) | low * |
| pRB401B | BL21(DE3)(plysS) (RB401C) | 12% |
| pRB401A | BL21 (RB401D) | 15% |

* not detectable by Commassie Blue Staining but detectable by Western Blot.

The results indicate that the T7 expression system in E. coli BL21(DE3)(pLysS) or $\lambda P_L$ expression system in E. coli BL21 is especially efficient for the expression of the DNA sequence coding for Met-Arg-Met-MPF-4 as the Met-Arg-Met-MPF-4 polypeptide was produced as about 15% of the total cell protein. This is in contrast to the expression of the Met-MPF-4 gene which was expressed at low levels using both expression systems and in all transformed cell types.

## EXAMPLE 3

## Expression of Met-Arg-Met-PF-4

## A. Construction of Plasmid pRB402A/$\lambda$ $P_L$ Expression System

Plasmid pRB402A contains a DNA sequence coding for PF-4 having an N-terminal amino acid sequence Met-Arg-Met under control of the $\lambda$ $P_L$ promoter. Plasmid pRB402A was constructed as follows and as shown in Figure 2.

About 20 μg of the plasmid pRB400A DNA was added to 20 μl of 10X NdeI buffer (100 mM NaCl, 50 mM Tris-HCl (pH 7.5), 10 mM MgCl$_2$, 1 mM dithioerythritol (DTE), 100 μg/ml BSA), 5 μl (40 units) NdeI restriction enzyme and 175 μl water. After mixing the reaction was incubated at 37°C for 2 hours. The DNA was precipitated by adding sodium acetate to a final concentration of 0.3M, adding three volumes of ethanol, mixing and chilling to -70°C, and centrifuging. The DNA pellet was washed with 70% ethanol (1 ml); dried; and dissolved in 20 μl of 10X SpeI buffer (100 mM NaCl, 50 mM Tris-HCl (pH 7.5), 10 mM MgCl$_2$, 1 mM DTE, 100 μg/ml BSA), 4 μl (40 units) of SpeI restriction enzyme, and 176 μl water. After gentle mixing, the reaction was incubated at 37°C for 2 hours. The DNA was again precipitated with three volumes of ethanol as above and electrophoresed on a 1% low melting agarose gel. The large (5.95 Kb) DNA fragment was sliced from the gel and the DNA was recovered by melting the agarose and passing through an Elutip-d column. After precipitation and drying, the purified DNA was stored in 30 μl of 10 mM Tris-HCl (pH 8.0).

Oligonucleotide coding for a Met-Arg-Met cleavable peptide leader sequence for PF-4 were synthetically prepared. Four single stranded oligonucleotides designated MPFL-3, MPFL-4, MPFL-5 and MPFL-6, as shown below:

```
MPFL-3 (SEQ ID NO:11):
        5' - TCC CAG GTC CGT CCG CGT CAC ATC A  - 3'


MPFL-4 (SEQ ID NO:12):
        5' - CTAGT GAT GTG ACG CGG ACG GACCT  - 3'


MPFL-5 (SEQ ID NO:15):
        5' - TATG CGT ATG GAA GCT GAA GAA GAT GGT GAC CTG

                CAG TGC CTG TGT GTG AAG ACC ACC - 3'


MPFL-6 (SEQ ID NO:16):
        5' - GGGA GGT GGT CTT CAC ACA CAG GCA CTG CAG GTC ACC

                ATC TTC TTC AGC TTC CAT ACG CA  - 3'
```

were prepared on a 380B DNA synthesizer and purified by polyacrylamide gel electrophoresis.

About 200 picomoles of oligonucleotides were phosphorylated, annealed and ligated, as described previously to form the following linker:

```
5'- TATG CGT ATG GAA GCT GAA GAA GAT GGT
3'     AC GCA TAC CTT CGA CTT CTT CTA CCA

    GAC CTG CAG TGC CTG TGT GTG
    CTG GAC GTC ACG GAC ACA CAC

    AAG ACC ACC TCC CAG GTC
    TTC TGG TGG AGG GTC CAG

    CGT CCG CGT CAC ATC A      - 3' (SEQ ID NO:41)
    GCA GGC GCA GTG TAG TGA TC  - 5' (SEQ ID NO:42)
```

This synthetic linker codes for the Met-Arg-Met peptide leader sequence and the first 24 N-terminal amino acids of PF-4.

About 20 picomoles of this linker were mixed with 2.5 μl NdeI-SpeI digested pRB400A in a buffer containing 50 mM Tris-HCl (pH 7.6), 100 mM MgCl$_2$, 100 mM DTT, 800 μM ATP, and 3.0 units of T$_4$-DNA ligase. The reaction mixture was incubated at 4°C overnight and then transformed into E. coli K12RV308 as described previously. The desired transformant, E. coli RV308/pRB402A, was identified by sequence analysis of its plasmid DNA. The cells were grown and protein expression was induced and quantitated as described previously. A partial restriction site map of plasmid pRB402A is presented in Figure 2.

About 0.5 μg of plasmid pRB402A was transformed into E. coli BL21 cells to form RB402D.

**B. Construction of pRB402B/T7 Expression System**

Plasmid pRB402B contains a DNA sequence coding for Met-Arg-Met-PF-4 under the control of the T$_7$ promoter. Plasmid pRB402B was constructed as follows and as shown in Figure 3.

Plasmid pHS564 was digested with NdeI and BamHI as described in Example 1. A 3.72 Kb fragment corresponding to the NdeI/BamHI plasmid digest was purified and stored in 10mM Tris-HCl (pH 7.6).

Plasmid pRB402A was digested with NdeI and BamHI as follows. About 20 μg of this plasmid pRB402A DNA was added to 20 μl of 10X NdeI buffer (100 mM NaCl, 50 mM Tris-HCl (pH 7.5), 10 mM MgCl$_2$, 1 mM Dithioerythritol (DTE), 100 μg/ml BSA), 5 μl (40 units) NdeI restriction enzyme and 175 μl water. After mixing,

the reaction was incubated at 37°C for 2 hours. The DNA was precipitated by adding sodium acetate to a final concentration of 0.3M, adding three volumes of ethanol, mixing and chilling to -70°C, and centrifuging. The DNA pellet was washed with 70% ethanol (1 ml); dried; and dissolved in 20 μl of 10X <u>Bam</u>HI buffer (100 mm NaCl, 10 mM Tris-HCl (ph 8.0), 10 mM MgCl$_2$, 1 mM 2-mercaptoethanol, 100 μg/ml BSA), 2 μl (25 units) of <u>Bam</u>HI restriction enzyme, and 178 μl water. After gentle mixing, the reaction was incubated at 37°C for 2 hours. The DNA was again precipitated with three volumes of ethanol as above and electrophoresed on a 1% low melting agarose gel. The desired DNA fragment coding for Met-Arg-Met-PF-4 corresponding to about 226 bp was sliced from the gel and then DNA was recovered by melting the agarose and passing through an Elutip-d column. After precipitation and drying, the purified DNA was stored in 30 μl of 10 mM Tris-HCl (pH 8.0).

About 2.5 μl of the <u>Nde</u>I-<u>Bam</u>HI vector DNA fragment of pHS564 was mixed with 12 μl of the purified Met-Arg-Met-PF-4 gene fragment from above, 4 μl of 10 mM ATP, 0.5 μl of 1 M DTT, 5 μl of 10X Ligase Buffer, 26 μl of water, and 0.5 μl (3.0 units) of T$_4$-DNA ligase. The reaction mixture was incubated at 4°C for 16 hours. The ligated mixture was diluted with 50 μl of 10 mM Tris-HCl (pH 7.6) and 3 μl of 1 M CaCl$_2$ and then subsequently transformed into <u>E. coli</u> BL21(DE3) to form RB402B and BL21(DE3)(pLysS) to form RB402C, as described previously.

The desired transformants were detected by DNA sequence analysis. The cells were grown and protein expression was induced and quantitated as described previously. The polypeptide sequence of Met-Arg-Met-PF-4 is shown in SEQ ID NO:18.

## C. Detection of Expression of Met-Arg-Met-PF-4

The expression of Met-Arg-Met-PF-4 was detected by electrophoresis of transformed cell lysates on a 15% SDS polyacrylamide gel. Gels were stained with Commassie Brilliant Blue or by Western blotting. Expression levels of the recombinant polypeptides were quantitated by scanning densitometry. The results are shown in Table II.

**TABLE II**

| Plasmid | Strain | Expression Level (% of Total Protein) |
|---------|--------|--------------------------------------:|
| pRB402A | RV308 (RB402A) | 8% |
| pRB402B | BL21(DE3) (RB402B) | 20% |
| pRB402B | BL21(DE3)(pLysS)(RB402C) | low |
| pRB402A | BL21 (RB402D) | 20% |

The results shown in Table II indicate that Met-Arg-Met-PF-4 was expressed at high levels (i.e., about 15-20% of total protein by the cells) in the BL21 cells. These results are similar to those obtained for Met-Arg-Met-MPF-4.

EXAMPLE 4

## Expression of Met-Arg-Met-PF-4*

## 1. Construction of Plasmid pRB403A/λ P$_L$ Promoter Expression System

The plasmid pRB403A includes a DNA sequence coding for a polypeptide having the following amino acid sequence (SEQ ID NO:22)

```
Met-Arg-Met-Glu-Ala-Glu-Glu-Asp-Gly-Asp-
Leu-Gln-Cys-Leu-Cys-Val-Lys-Thr-Thr-Ser-
Gln-Met-MPF-4
```

under the control of the λ P$_L$ promoter. Plasmid pRB403A was constructed as follows and as shown in Figure 2.

About 20 µg of plasmid pRB400A DNA was added to 20 µl of 10X Ndel buffer (100 mM NaCl, 50 mM Tris-HCl (pH 7.5), 10 mM MgCl₂, 1 mM Dithioerythritol, 100 µg/ml BSA), 5 µl (40 units) Ndel restriction enzyme and 175 µl water. After mixing, the reaction was incubated at 37°C for 2 hours. The DNA was precipitated by adding sodium acetate to a final concentration of 0.3M, adding three volumes of ethanol, mixing and chilling to -70°C, and centrifuging. The DNA pellet was washed with 70% ethanol (1 ml); dried; and dissolved in 20 µl of 10X SpeI buffer (100 mM NaCl, 50 mM Tris-HCl (pH 7.5), 10 mM MgCl₂, 1 mM DTE, 100 µg/ml BSA), 4 µl (40 units) of SpeI restriction enzyme, and 176 µl water. After gentle mixing, the reaction was incubated at 37°C for 2 hours. The DNA was again precipitated with three volumes of ethanol as above and electrophoresed on a 1% low melting agarose gel. The large (5.95 Kb) DNA fragment was sliced from the gel and the DNA was recovered by melting the agarose and passing through an Elutip-d column. After precipitation and drying, the purified DNA was stored in 30 µl of 10 mM Tris-HCl (pH 8.0).

Synthetic oligonucleotide linkers coding for the first 30 N-terminal amino acids of Met-Arg-Met-PF-4* were prepared. Four single stranded oligonucleotides corresponding to MPFL-5, MPFL-6, MPFL-9, MPFL-10 below:

```
MPFL-5  (SEQ ID NO:15):
     5' - TATG CGT ATG GAA GCT GAA GAA GAT GGT GAC CTG

           CAG TGC CTG TGT GTG AAG ACC ACC - 3'



MPFL-6 (SEQ ID NO:16):
     5' - GGGA GGT GGT CTT CAC ACA CAG GCA CTG CAG GTC ACC

           ATC TTC TTC AGC TTC CAT ACG CA  - 3'



MPFL-9 (SEQ ID NO:19):
     5'- TCCCAGATGTCCCAGGTCCGTCCGCGTCACATCA - 3'



MPFL-10 (SEQ ID NO:20):
       5'- CTAGTGATGTGACGCGGACCTGGGACATCT - 3'
```

were synthesized on a 380B DNA synthesizer and purified by gel electrophoresis. The oligonucleotides were phosphorylated, annealed and ligated as described in Example 1 to form the following oligonucleotide:

```
5'- T ATG CGT ATG GAA GCT GAA GAA GAT
3'      AC GCA TAC CTT CGA CTT CTT CTA

   GGT GAC CTG CAG TGC CTG TGT GTG
   CCA CTG GAC GTC ACG GAC ACA CAC

   AAG ACC ACC TCC CAG ATG TCC
   TTC TGG TGG AGG GTC TAC AGG

   CAG GTC CGT CCG CGT CAC ATC A      3' (SEQ ID NO:43)
   GTC CAG GCA GGC GCA GTG TAG TGATC  5' (SEQ ID NO:44)
```

This synthetic oligonucleotide linker encodes the first 30 N-terminal amino acids of Met-Arg-Met PF-4*. The polypeptide sequence of Met-Arg-Met-PF-4* is shown in SEQ ID NO:22.

About 15 pmoles of this linker was mixed with 2.5 µl of Ndel-SpeI digested pRB400A in a buffer containing 50 mM Tris-HCl (pH 7.6), 100 mM MgCl₂, 100 mM DTT, 800 µM ATP, and 3.6 units of T₄-DNA ligase. The reaction mixture was incubated at 4°C overnight and then transformed into E. coli K12RV308 as described previously. The desired transformant, E. coli, K12RV308/pRB403A, was identified by sequence analysis of its plasmid DNA. The cells were grown and protein expression was induced and quantitated as described previously. A partial restriction site map of plasmid pRB403 is presented in Figure 2. E. coli strain K12RV308 harboring the plasmid pRB403A was deposited with the NRRL on _____(date) and given Accession No. NRRLB-21159.

About 0.5 µg of pRB403A was transformed into E.coli BL21 cells to form RB403D.

## B. Construction of Plasmid pRB403B/T7 Expression System

The plasmid pRB403B contains a DNA sequence coding for Met-Arg-Met-PF-4* having the amino acid sequence shown in SEQ ID NO:22 under the control of the $T_7$ promoter. Plasmid pRB403B was constructed as follows and as shown in Figure 3.

Plasmid pHS564 was digested with NdeI and BamHI as described in Example 1. A 3.72 Kb fragment corresponding to the NdeI/BamHI plasmid digest was purified and stored in 10 mM Tris-HCl (pH 7.6).

Plasmid pBR403A was digested with NdeI and BamHI as follows. About 20 µg of plasmid pRB403A DNA was added to 20 µl of 10X NdeI buffer (100 mM NaCl, 50 mM Tris-HCl (pH 7.5), 10 mM MgCl$_2$, 1 mM Dithioerythriotol, 100 µg/ml BSA), 5 µl (40 units) NdeI restriction enzyme and 175 µl water. After mixing, the reaction was incubated at 37°C for 2 hours. The DNA was precipitated by adding sodium acetate to a final concentration of 0.3M, adding three volumes of ethanol, mixing and chilling to -70°C, and centrifuging. The DNA pellet was washed with 70% ethanol (1 ml); dried; and dissolved in 20 µl of 10X BamHI buffer (100 mm NaCl, 10 mM Tris-HCl (ph 8.0), 10 mM MgCl$_2$, 1 mM 2-mercaptoethanol, 100 µg/ml BSA), 2 µl (25 units) of BamHI restriction enzyme, and 178 µl water. After gentle mixing, the reaction was incubated at 37°C for 2 hours. The DNA was again precipitated with three volumes of ethanol as above and electrophoresed on a 1% low melting agarose gel. The desired DNA fragment coding for Met-Arg-Met-PF-4* corresponding to about 235 bp was sliced from the gel and then DNA was recovered by melting the agarose and passing through an Elutip-d column. After precipitation and drying, the purified DNA was stored in 30 µl of 10 mM Tris-HCl (pH 8.0).

About 2.5 µl of the NdeI-BamHI vector DNA fragment of pHS564 was mixed with 12 µl of the purified Met-Arg-Met-PF-4* gene fragment from above, 4 µl of 10 mM ATP, 0.5 µl of 1 M DTT, 5 µl of 10X Ligase Buffer, 26 µl of water, and 0.5 µl (3-0 units) of $T_4$-DNA ligase. The reaction mixture was incubated at 4°C for 16 hours. The ligated mixture was diluted with 50 µl of 10 mM Tris-HCl (pH 7.6) and 3 µl of 1 M CaCl$_2$ and then subsequently transformed into E. coli BL21(DE3) to form RB403B and E. coli BL21(DE3)(pLysS) cells to form RB403C, respectively.

The desired transformants were detected by DNA sequence analysis. The cells were grown and protein expression was induced and quantitated as described previously.

## C. Detection of Expression of Met-Arg-Met-PF-4*

The expression of Met-Arg-Met-PF-4* genes was detected by electrophoresis of transformed cell lysates on 15% SDS-PAGE. Gels were stained with Commassie Brilliant Blue or by Western blotting. The results are shown in Table III.

**TABLE III**

| Plasmid | Strain | Expression Level (% of Total Protein) |
|---------|--------|--------------------------------------:|
| pRB403A | RV308 (RB403A) | low |
| pRB403B | BL21(DE3) (RB403B) | N.D |
| pRB403A | BL21 (RB403D) | 20% |

The results show that the $\lambda P_L$ expression system in E. coli strain BL21 resulted in a highly efficient production of recombinant Met-Arg-Met-PF-4* (up to 20% of total cell protein). These results are similar to those obtained with Met-Arg-Met-MPF-4, and Met-Arg-Met-PF-4.

**EXAMPLE 5**

**Purification, Biochemical Characterization, and Biological Activity of** *E. coli* **Derived** <u>Recombinant</u> <u>Modified Platelet Factor-4 (rMPF-4)</u>

**A.** <u>Cell Lysis and Protein Extraction</u>

*E. coli* pellet including recombinant Met-Arg-Met-MPF-4 was obtained from 5 liters of fermentation broth (15 g wet weight) and was suspended in 150 ml of lysis buffer consisting of 50 mM Tris, pH 7.6, 5 mM EDTA, 0.5% Triton X-100, 2 mg lysozyme/g cell pellet, 5 units DNAse/g cell pellet, and 0.2 mM phenyl methionyl sulfonyl fluoride (PMSF). Cells were lysed by sonication at 4°C using a Branson Sonifier cell disrupter. Sonication was repeated four times at 2 minute intervals. Alternatively, cell pellet was suspended in 7 M guanidinium hydrochloride. Cell lysate was then centrifuged at 10,000 X.g for 40 minutes. The supernatant was further clarified by another centrifugation at 10,000 X g for 40 minutes. The precipitates from the two centrifugation steps were discarded. The majority of recombinant MPF-4 was present in the supernatant, as determined by polyacrylamide gel electrophoresis and Western blot analysis.

**B.** <u>Heparin-Sepharose Chromatography</u>

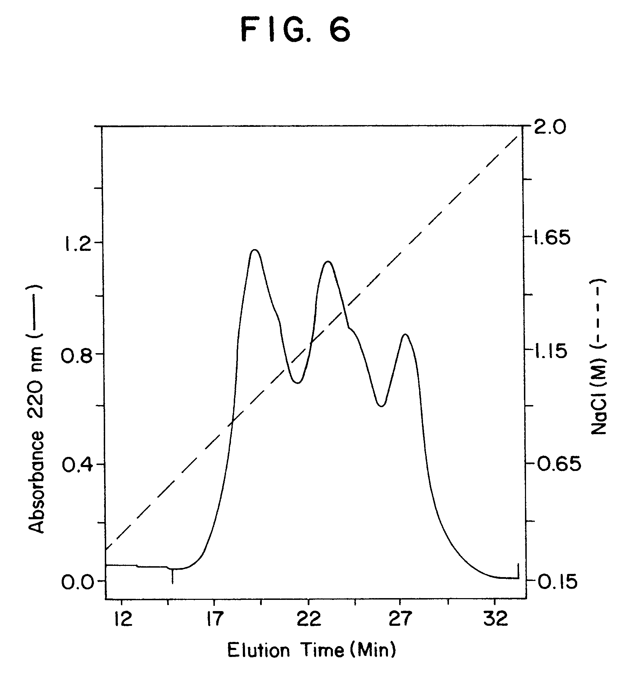

An aliquot of the protein extract was applied to a Heparin-Sepharose column (Pharmacia 5.0 ml HiTrap-Heparin) pre-equilibrated with Dulbecco's Phosphate Buffered Saline. The column was washed and then eluted with a 40 minute linear gradient of PBS (buffer A) and 2.0 M NaCl in PBS (buffer B). The flow rate was 1.0 ml/minute. Alternatively, the column was equilibrated with 25 mM sodium phosphate buffer, pH 7.5, containing 1 M urea and 1 mM EDTA. The column was eluted with the 2 mol NaCl gradient buffers containing 1 M urea. An example of the elution profile from heparin sepharose column is shown in Figure 6. The fractions containing recombinant Met-Arg-Met-MPF-4 as determined by Western blot analysis using PF-4 antibodies were pooled. Pooled sample was analyzed by Western immunoblotting, amino acid sequencing and inhibition of endothelial cell proliferation.

**C. Reverse Phase C-4 HPLC**

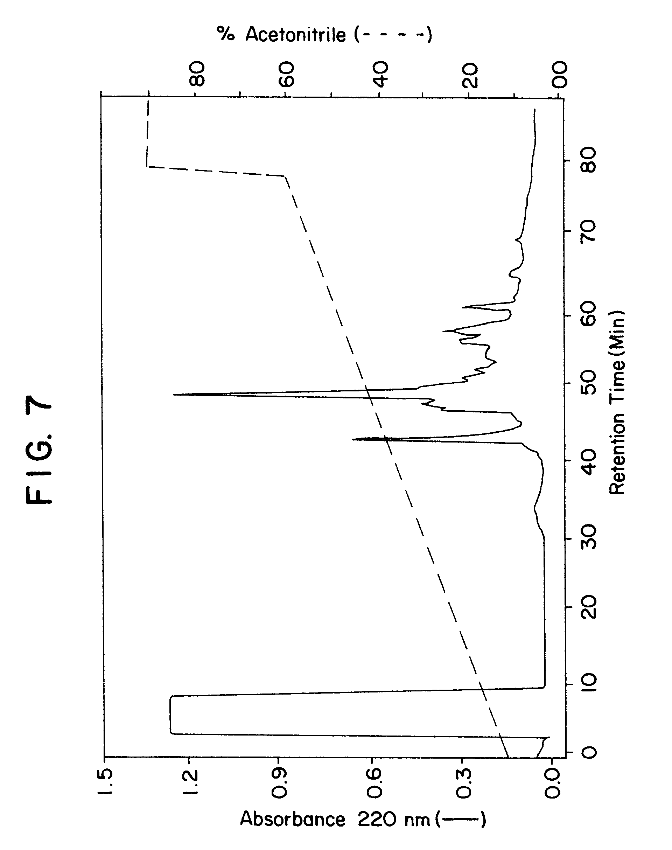

Heparin sepharose purified MPF-4 was fractionated by C-4 reverse phase column chromatography. A reverse phase C-4 column (25 x 0.4 cm, Vydac, Inc.) was equilibrated with solvent A containing 10% acetonitrile, 89.95% $H_2O$, and 0.05% trifluroacetic acid (TFA). Recombinant Met-Arg-Net-MPF-4 fractions were applied to the column at a flow rate of 1.0 ml/minute. The column was washed with solvent A. The bound proteins were then eluted with a linear gradient of solvent A and solvent B (59.95% acetonitrile, 39.95% $H_2O$ containing 0.05% TFA). The flow rate was 1.0 ml/min. An example of the elution profile from the reversed phase column is shown in Figure 7. Aliquots of individual fractions were concentrated and analyzed by SDS polyacrylamide gel electrophoresis. Recombinant Met-Arg-Met-MPF-4 containing fractions were pooled and lyophilized.

**D.** <u>Cyanogen Bromide Cleavage</u>

The lyophilized pellet was suspended into 70% formic acid (10 mg protein/ml formic acid). CNBr was added to the suspension to a final concentration of 3 M. The CNBr reaction was conducted for 24 hours at room temperature in the dark. The reaction mixture was then diluted 1:3 with distilled water and lyophilized. The lyophilized powder was suspended in reverse phase column buffer A. The presence of MPF-4 was confirmed by sodium dodecyl sulfate-polyacrylamide gel electrophoresis (SDS-PAGE) 16% Tris/Tricine gel, and Western immunoblot using anti-PF-4 antibody.

**E.** <u>Reverse Phase Chromatography</u>

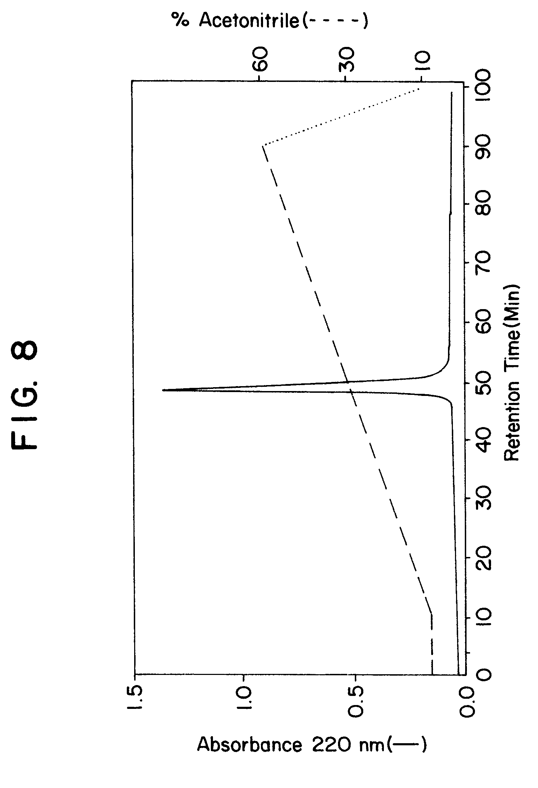

CNBr cleaved MPF-4 was further purified by C-4 reverse phase column chromatography. A reverse phase C-4 column (Vydac, Inc.) was equilibrated with solvent A containing 10% acetonitrile, 89.95% $H_2O$ and 0.05% TFA. MPF-4 fractions were applied to the column at flow rate of 1.0 ml/minute. The column was washed with solvent A. The bound proteins were then eluted with linear gradient of solvent A and solvent B (59.95% acetonitrile, 39.95% $H_2O$ containing 0.05% TFA). The flow rate was 1.0 ml/min. An example of the elution profile from the reversed phase column is shown in Figure 8. Aliquots of individual fractions were concentrated and analyzed by polyacrylamide gel electrophoresis. Recombinant MPF-4 containing fractions were pooled and lyophilized.

## F. Polyacrylamide Gel Electrophoresis

Proteins were analyzed by SDS-PAGE. Approximately 2-10 μg protein/lane were loaded onto 16% Tris-Tricine mini-gels (Novex, Inc.) and run at 125 volts constant. Gels were transferred to coomassie staining solution and destained for visualization.

## G. Western Blot Analysis

Following SDS gel electrophoresis, proteins were transferred onto Western, 0.2 micron (Schleicher and Schuell, Inc.) filters. Transfer was conducted at 50 volts for one hour. Transferred proteins were blocked by incubation in 5% Casein, Tris-HCl Buffered Saline (TBS) (25 mM Tris, 0.9% NaCl, pH 7.4) at room temperature for one hour. The blot was incubated with 1:200 diluted TBS primary polyclonal goat anti-PF-4 (Atlantic Antibodies, Inc.) for one hour and then washed 3 times by incubation in TBs for 10 minutes. The blot was then incubated with a 1:200 diluted secondary antibody, rabbit anti-goat IgG alkaline phosphatase conjugated (Pierce, Inc.) for a minimum of one hour. Secondary antibody was washed off, as in the previous wash step, and the blot was developed using an alkaline phosphatase conjugate substrate kit (BioRad, Inc.).

## H. Amino Acid N-Terminus Sequence Conformation

SDS-PAGE Tris-Tricine electrophoresed proteins were transferred onto 0.2 micron PVDF membrane (Schleicher and Schuell, Inc.) according to the Western blot procedure as described above. The PVDF membrane was stained with coomassie for 5 minutes and then destained in 50% methanol until background diminished. Protein bands were cut from the PVDF and sequenced by automatic protein sequencer using standard methods.

## EXAMPLE 6

## Endothelial Cell Proliferation Inhibition Assay

Bovine heart endothelial cells were obtained (American Type Culture Collection; Rockville, MD 20852) for use in the assay. The endothelial cells were seeded into 24 well tissue culture plates at a cell density of 10,000 cells/well in Dulbecco's modified Eagle Medium (GIBCO, Grand Island, NY) supplemented with 10% bovine serum albumin, 100 U/ml penicillin, 100 μg/ml streptomycin, 2 mM L-glutamine, and 20 ng/ml fibroblast growth factor. Immediately after seeding the cells, various amounts of recombinant Met-Arg-Met-MPF-4, or recombinant MPF-4 were added to individual wells. MPF-4 was tested at final concentrations ranging from 0.01 to 30 μg/ml. Met-Arg-Met-MPF-4 was tested for biological activity after heparin-sulfate chromatography. Recombinant MPF-4 was tested for biological activity after cleavage and purification by reverse phase HPLC. The endothelial cell cultures were allowed to grow for 4 days at 37°C, 5% $CO_2$ in a humidified tissue culture incubator. The cultures were then harvested individually by trypsinization and counted using a Zm-Coulter Counter (Coulter Electronics, Inc., Opa Locka, FL 33054).

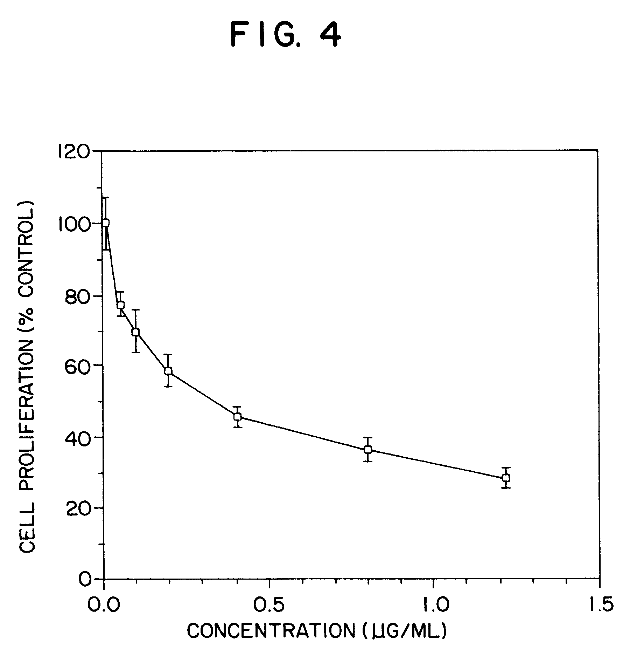

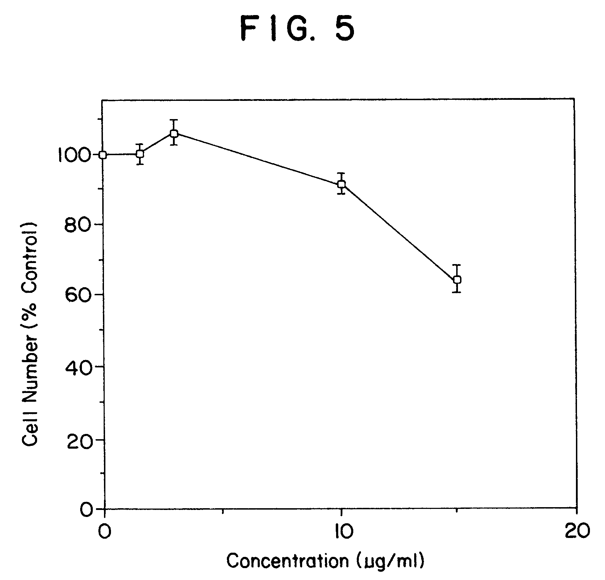

The results of the biological activity of Met-Arg-Met-MPF-4 after heparin sulfate chromatography are shown in Figure 4. The results show inhibition of endothelial cell proliferation by Met-Arg-Met-MPF-4 in a dose response manner. After cleavage and subsequent purification of recombinant MPF-4, it was tested for biological activity and the results are shown in Figure 5. Recombinant MPF-4 inhibited endothelial cell proliferation in a dose response manner and retained significant biological activity.

## EXAMPLE 7

## Expression of Met-Arq-Met-PF-4[†]

A plasmid encoding Met-Arg-Met-PF-4[†] can be constructed using methods described previously. The polypeptide sequence of Met-Arg-Met-PF-4[†] is shown in SEQ ID NO:27.

A DNA sequence coding for Met-MPF-4 can be obtained as described in Example 1, or derived from a DNA sequence coding for PF-4. A synthetic DNA sequence coding for Met-MPF-4 can be prepared as shown in Figure 1 and described in Example 1. This sequence is present in plasmid pRB400A ($\lambda P_L$ promoter) or pRB400B ($T_7$ promoter).

The oligonucleotide linkers can be prepared synthetically to code for a cleavable peptide leader sequence having the following amino acid sequence (SEQ ID NO:45):

```
    Met-Arg-Met-Glu-Ala-Glu-Glu-Asp-Gly-Asp-Leu-Gln-Cys-
    Leu-Cys-Val-Lys-Thr-Met.
```

The oligonucleotide linker can have the DNA sequence corresponding to MPFL-11 and MPFL-12 as shown below:

```
MPFL-11 (SEQ ID NO:24):
    5'- T ATG CGT ATG GAA GCT GAA GAA GAT GGT GAC CTG CAG TGC CTG
            TGT GTG AAG ACC ATG - 3'


 MPFL-12 (SEQ ID NO:25):
    5'- G GGA CAT GGT CTT CAC ACA CAG GCA CTG CAG GTC ACC ATC TTC
            TTC AGC TTC CAT ACG CA - 3'
```

The oligonucleotide linker can be phosphorylated, annealed, and ligated as described in Example 1 to form the following oligonucleotide:

```
    5'- TATG CGT ATG GAA GCT GAA GAA GAT GGT GAC CTG CAG
    3'     AC GCA TAC CTT CGA CTT CTT CTA CCA CTG GAC GTC

    TGC CTG TGT GTG AAG ACC ATG          - 3' (SEQ ID NO:46)
    ACG GAC ACA CAC TTC TGG TAC AGG G  -  5' (SEQ ID NO:47)
```

About 15 picomoles of this linker and 15 moles phosphorylated linker consisting of MPFL-3 and MPFL-4 can be mixed with an NdeI/SpeI restriction fragment from pRB400A and ligated as described previously. The ligated mixture can be transformed into E. coli RV308 or E. coli BL21 cells. Transformed cells can be selected and grown in (culture overnight. Expression of Met-Arg-Met-PF-4[†] can be induced and quantitated as described previously.

All publications and patent applications in this specification are indicative of the level of ordinary skill in the art to which this invention pertains. All publications and patent applications are herein incorporated by reference to the same extent as if each individual publication or patent application was specifically and individually indicated by reference.

While the present invention has been described in connection with the preferred embodiment thereof, it will be understood many modifications will be readily apparent to those skilled in the art, and this application is intended to cover any adaptations or variations thereof. It is manifestly intended this invention be limited only by the claims and equivalents thereof.

## Claims

1. A recombinant DNA molecule comprising a DNA having a sequence that encodes X-MPF-4, wherein X is a cleavable leader sequence.

2. A recombinant DNA molecule according to claim 1, wherein X is Met-Arg-Met or Met-Arg.

3. A recombinant DNA molecule according to claim 1, wherein X is a polypeptide having a sequence comprising:

```
        Met-Arg-Met-Glu-Ala-Glu-Glu-Asp-Gly-Asp-Leu-
        Gln-Cys-Leu-Cys-Val-Lys-Thr-Thr-Ser-Gln-Met
        (SEQ ID NO:5)
```

or

```
        Met-Arg-Met-Glu-Ala-Glu-Glu-Asp-Gly-Asp-Leu-
        Gln-Cys-Leu-Cys-Val-Lys-Thr-Met (SEQ ID
        NO:6).
```

4. A recombinant DNA molecule comprising a DNA having a sequence that encodes X-PF-4 or X-CPF-4-A, wherein X is a cleavable peptide leader sequence.

5. A recombinant DNA molecule according to claim 4, wherein X is Met-Arg-Met or Met-Arg. ,

6. A recombinant DNA molecule comprising a DNA having a sequence that encodes a polypeptide MPF-4.

7. A recombinant DNA vector comprising the DNA sequence of claim 1 operably linked to transcriptional and translational control regions in the vector, said vector capable of driving the expression of the DNA sequence of claim 1 when transformed into a suitable host cell.

8. A recombinant DNA vector comprising a DNA having a sequence of claim 4 operably linked to transcriptional and translational control regions in the vector, said vector capable of driving the expression of the DNA sequence of claim 4 when transformed into a suitable host cell.

9. A recombinant DNA vector according to claim 7, wherein the transcriptional control regions comprise the λ $P_L$ promoter.

10. A recombinant DNA vector according to claim 7, wherein the transcriptional control regions comprise the $T_7$ promoter.

11. A host cell transformed with the vector of claim 7.

12. A host cell transformed with the vector of claim 8.

13. A transformed E. coli strain according to claim 11 having the characteristics of NRRLB-21158.

14. A transformed E. coli strain according to claim 11 having the characteristics of NRRLB-21157.

15. A transformed E. coli strain according to claim 11 having the characteristics of NRRLB-21159.

16. A host cell according to claim 7, wherein the host cell is selected from the group consisting of E. coli K12RV308, E. coli BL21(DE3), E. coli BL21(DE3)(pLysS), and E. coli BL21.

17. An isolated polypeptide having a sequence X-MPF-4 wherein X is a cleavable leader sequence.

18. An isolated polypeptide according to claim 17, wherein the X is selected from:

```
Met;

Met-Arg;

Met-Arg-Met-;

Met-Arg-Met-Glu-Ala-Glu-Glu-Asp-Gly-Asp-Leu-Gln-
Cys-Leu-Cys-Val-Lys-Thr-Thr-Ser-Gln-Met (SEQ ID
NO:5); or

Met-Arg-Met-Glu-Ala-Glu-Glu-Asp-Gly-Asp-Leu-Gln-
Cys-Leu-Cys-Val-Lys-Thr-Met (SEQ ID NO:6).
```

19. An isolated polypeptide having a sequence X-PF-4 or X-CPF-4, wherein X is a cleavable leader sequence.

20. An isolated polypeptide according to claim 19, wherein X is Met-Arg or Met-Arg-Met.

21. A pharmaceutical composition comprising:
    a polypeptide according to claim 18 in admixture with a pharmaceutically acceptable diluent.

22. A method of inhibiting angiogenesis which comprises administering an effective amount of the composition of claim 21.

23. A pharmaceutical composition comprising
a polypeptide according to claim 19 in admixture with a pharmaceutically acceptable diluent.

24. A method of inhibiting angiogenesis which comprises administering an effective amount of the composition of claim 23.

# FIG. 1A

```
                              E   S           M T                          S
                      C       Bc SAa          a s                          a
                   Av NT   N   FsoHcvu    A Te p     SBM               BuD
                   li sa   d   iaRgra9    c hI 4     pfn               c3p
                   uJ pq   e   nJIaFI6    i aI 5     eal               lAn
                   II VI   I   IIIIIII    I II I     III               III
                   /  /        / / /                                  /
              AGCTTCGAACATATGTCCCAGGTCCGTCCGCGTCACATCACTAGTCTGGAGGTGATCAAG
         1    ----+---------+---------+---------+---------+---------+ 60
              GCTTGTATACAGGGTCCAGGCAGGCGCAGTGTAGTGATCAGACCTCCACTAGTTC

              MetSerGlnValArgProArgHisIleThrSerLeuGluValIleLys


              H       S
         BCaA      Ra                                          M      T
         svevHMABsu                                            b  A   s
         riIapscpr9                                            o  p   p
         FJIIhpimI6                                            I  o   E
         IIIIIIIIII                                            I  I   I
         // // ///
         GCCGGTCCGCACTGCCCGACTGCCCAACTGATTGCCACTCTGAAGAATGGTCGTAAAATT
     61  ---------+---------+---------+---------+---------+---------+ 120
         CGGCCAGGCGTGACGGGCTGACGGGTTGACTAACGGTGAGACTTCTTACCAGCATTTTAA

         AlaGlyProHisCysProThrAlaGlnLeuIleAlaThrLeuLysAsnGlyArgLysIle
```

EP 0 682 114 A2

# FIG. 1B

EP 0 682 114 A2

```
                                        B
                                        s
     EE        S              N         p
     cc  S  ABa      C  C  CBB  s  1                          H
     oo  c  vsu      v  a  vssA  p  4  RF        M        M    i     MA  P
     5R  r  ap9      i  c  ippc  B  0  sa        s        l    n     sl  l
     7I  F  IG6      R  8  JMWi  I  7  au        e        y    f     ew  e
     II  I  III      I  I  IIII  I  I  II        I        I    I     II  I
            //
     TGCCTGGACCTGCAAGCCCCGCTGTACAAGAAAATCATTAAGAAACTTCTGGAGTCTTAA
121  ---------+---------+---------+---------+---------+---------+ 180
     ACGGACCTGGACGTTCGGGGCGACATGTTCTTTTAGTAATTCTTTGAAGACCTCAGAATT

     CysLeuAspLeuGlnAlaProLeuTyrLysLysIleIleLysLysLeuLeuGluSerEnd


           S
     BB  Na       ET
     asDlu  ABAcs
     mtpa3  pplop
     HYnIA  omwRE
     IIIVI  IIIII
       / //      //
     TAGGATCCGAATTC
181  ----------      194
     ATCCTAGGC

     EndAspPro
```

# FIG. 2A

# FIG. 2B

EP 0 682 114 A2

# FIG. 3

# F I G. 4

# F I G. 5

# FIG. 6

FIG. 7

FIG. 8

## SEQUENCE LISTING

(1) GENERAL INFORMATION:

    (i) APPLICANT: ELI LILLY AND COMPANY
        (B)   STREET:      Lilly Corporate Center
        (C)   CITY:        Indianapolis
        (D)   STATE:       Indiana
        (E)   COUNTRY:     United States of America
        (F)   ZIP:         46285

    (ii) TITLE OF INVENTION: Expression of Recombinant MPF-4, CPF-4, PF-4 and Precursor Compounds

    (iii) NUMBER OF SEQUENCES: 47

    (iv) CORRESPONDENCE ADDRESS:
        (A)  ADDRESSEE:   C. M. Hudson
        (B)  STREET:      Erl Wood Manor
        (C)  CITY:        Windlesham
        (D)  STATE:      Surrey
        (E)  COUNTRY:    United Kingdom
        (F)  ZIP:        GU20 6PH

    (v) COMPUTER READABLE FORM:
        (A)  MEDIUM TYPE: Floppy disk
        (B)  COMPUTER: Macintosh
        (C)  OPERATING SYSTEM: Macintosh 7.0
        (D)  SOFTWARE: Microsoft Word 5.1

(2) INFORMATION FOR SEQ ID NO:1:

    (i) SEQUENCE CHARACTERISTICS:
        (A)  LENGTH: 194 base pairs
        (B)  TYPE: nucleic acid
        (C)  STRANDEDNESS: single
        (D)  TOPOLOGY: linear

    (ii) MOLECULE TYPE: DNA (genomic)

    (vii) IMMEDIATE SOURCE:
        (B)  CLONE: Synthetic sequence coding for Met-MPF-4

    (ix) FEATURE:
        (A)  NAME/KEY: CDS
        (B)  LOCATION: 13..177

(xi) SEQUENCE DESCRIPTION: SEQ ID NO:1:

```
AGCTTCGAAC AT ATG TCC CAG GTC CGT CCG CGT CAC ATC ACT AGT CTG   48
              Met Ser Gln Val Arg Pro Arg His Ile Thr Ser Leu
               1               5                   10

GAG GTG ATC AAG GCC GGT CCG CAC TGC CCG ACT GCC CAA CTG ATT GCC 96
Glu Val Ile Lys Ala Gly Pro His Cys Pro Thr Ala Gln Leu Ile Ala
         15              20                  25

ACT CTG AAG AAT GGT CGT AAA ATT TGC CTG GAC CTG CAA GCC CCG CTG 144
Thr Leu Lys Asn Gly Arg Lys Ile Cys Leu Asp Leu Gln Ala Pro Leu
     30              35                  40

TAC AAG AAA ATC ATT AAG AAA CTT CTG GAG TCT TAATAGGATC CGAATTC 194
Tyr Lys Lys Ile Ile Lys Lys Leu Leu Glu Ser
45              50                  55
```

(2)  INFORMATION FOR SEQ ID NO:2:

        (i)  SEQUENCE CHARACTERISTICS:
             (A) LENGTH: 55 amino acids
             (B) TYPE: amino acid
             (D) TOPOLOGY: linear

        (ii) MOLECULE TYPE: protein

        (xi) SEQUENCE DESCRIPTION: SEQ ID NO:2:

```
Met Ser Gln Val Arg Pro Arg His Ile Thr Ser Leu Glu Val Ile Lys
1               5                   10                  15

Ala Gly Pro His Cys Pro Thr Ala Gln Leu Ile Ala Thr Leu Lys Asn
             20                  25                  30

Gly Arg Lys Ile Cys Leu Asp Leu Gln Ala Pro Leu Tyr Lys Lys Ile
         35                  40                  45

Ile Lys Lys Leu Leu Glu Ser
     50                  55
```

2)  INFORMATION FOR SEQ ID NO:3:

        (i)  SEQUENCE CHARACTERISTICS:
             (A) LENGTH: 70 amino acids
             (B) TYPE: amino acid
             (D) TOPOLOGY: linear

        (ii) MOLECULE TYPE: peptide

(vii) IMMEDIATE SOURCE:
        (B) CLONE: Human platelet factor-4 (PF-4)


    (xi) SEQUENCE DESCRIPTION: SEQ ID NO:3:

Glu Ala Glu Glu Asp Gly Asp Leu Gln Cys Leu Cys Val Lys Thr Thr
1               5                   10              15

Ser Gln Val Arg Pro Arg His Ile Thr Ser Leu Glu Val Ile Lys Ala
            20              25              30

Gly Pro His Cys Pro Thr Ala Gln Leu Ile Ala Thr Leu Lys Asn Gly
        35              40              45

Arg Lys Ile Cys Leu Asp Leu Gln Ala Pro Leu Tyr Lys Lys Ile Ile
    50              55              60

Lys Lys Leu Leu Glu Ser
65              70


(2) INFORMATION FOR SEQ ID NO:4:

        (i) SEQUENCE CHARACTERISTICS:
            (A) LENGTH: 54 amino acids
            (B) TYPE: amino acid
            (D) TOPOLOGY: linear

        (ii) MOLECULE TYPE: peptide

    (vii) IMMEDIATE SOURCE:
            (B) CLONE: Modified platelet factor-4 (MPF-4)

    (xi) SEQUENCE DESCRIPTION: SEQ ID NO:4:

Ser Gln Val Arg Pro Arg His Ile Thr Ser Leu Glu Val Ile Lys Ala
1               5                   10              15

Gly Pro His Cys Pro Thr Ala Gln Leu Ile Ala Thr Leu Lys Asn Gly
        20              25              30

Arg Lys Ile Cys Leu Asp Leu Gln Ala Pro Leu Tyr Lys Lys Ile Ile
        35              40              45

Lys Lys Leu Leu Glu Ser
        50

(2) INFORMATION FOR SEQ ID NO:5:

    (i) SEQUENCE CHARACTERISTICS:
        (A) LENGTH: 22 amino acids
        (B) TYPE: amino acid
        (D) TOPOLOGY: linear

    (ii) MOLECULE TYPE: peptide


    (xi) SEQUENCE DESCRIPTION: SEQ ID NO:5:

Met Arg Met Glu Ala Glu Glu Asp Gly Asp Leu Gln Cys Leu Cys Val
1               5                   10                  15

Lys Thr Thr Ser Gln Met
            20


(2) INFORMATION FOR SEQ ID NO:6:

    (i) SEQUENCE CHARACTERISTICS:
        (A) LENGTH: 19 amino acids
        (B) TYPE: amino acid
        (D) TOPOLOGY: linear

    (ii) MOLECULE TYPE: peptide


    (xi) SEQUENCE DESCRIPTION: SEQ ID NO:6:

Met Arg Met Glu Ala Glu Glu Asp Gly Asp Leu Gln Cys Leu Cys Val
1               5                   10                  15

Lys Thr Met


(2) INFORMATION FOR SEQ ID NO:7:

    (i) SEQUENCE CHARACTERISTICS:
        (A) LENGTH: 16 amino acids
        (B) TYPE: amino acid
        (D) TOPOLOGY: linear

    (ii) MOLECULE TYPE: peptide


    (xi) SEQUENCE DESCRIPTION: SEQ ID NO:7:

Glu Ala Glu Glu Asp Gly Asp Leu Gln Cys Leu Cys Val Lys Thr Thr
1               5                   10                  15

(2) INFORMATION FOR SEQ ID NO:8:

    (i) SEQUENCE CHARACTERISTICS:
        (A) LENGTH: 55 amino acids
        (B) TYPE: amino acid
        (D) TOPOLOGY: linear

    (ii) MOLECULE TYPE: peptide

    (vii) IMMEDIATE SOURCE:
        (B) CLONE: Polypeptide Met-MPF-4

    (xi) SEQUENCE DESCRIPTION: SEQ ID NO:8:

Met Ser Gln Val Arg Pro Arg His Ile Thr Ser Leu Glu Val Ile Lys
1            5                10            15

Ala Gly Pro His Cys Pro Thr Ala Gln Leu Ile Ala Thr Leu Lys Asn
          20              25            30

Gly Arg Lys Ile Cys Leu Asp Leu Gln Ala Pro Leu Tyr Lys Lys Ile
      35              40            45

Ile Lys Lys Leu Leu Glu Ser
   50            55

(2) INFORMATION FOR SEQ ID NO:9:

    (i) SEQUENCE CHARACTERISTICS:
        (A) LENGTH: 10 base pairs
        (B) TYPE: nucleic acid
        (C) STRANDEDNESS: single
        (D) TOPOLOGY: linear

    (ii) MOLECULE TYPE: DNA (genomic)

    (vii) IMMEDIATE SOURCE:
        (B) CLONE: Oligonucleotide MPFL-1

    (xi) SEQUENCE DESCRIPTION: SEQ ID NO:9:

TATGCGTATG   10

(2) INFORMATION FOR SEQ ID NO:10:

    (i) SEQUENCE CHARACTERISTICS:
        (A) LENGTH: 12 base pairs
        (B) TYPE: nucleic acid
        (C) STRANDEDNESS: single
        (D) TOPOLOGY: linear

    (ii) MOLECULE TYPE: DNA (genomic)

    (vii) IMMEDIATE SOURCE:
        (B) CLONE: Oligonucleotide MPFL-2

    (xi) SEQUENCE DESCRIPTION: SEQ ID NO:10:

GGGACATACG CA   12

(2) INFORMATION FOR SEQ ID NO:11:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (ii) MOLECULE TYPE: DNA (genomic)

    (vii) IMMEDIATE SOURCE:
        (B) CLONE: Oligonucleotide MPFL-3

    (xi) SEQUENCE DESCRIPTION: SEQ ID NO:11:

TCCCAGGTCC GTCCGCGTCA CATCA    25

(2) INFORMATION FOR SEQ ID NO:12:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (ii) MOLECULE TYPE: DNA (genomic)

    (vii) IMMEDIATE SOURCE:
        (B) CLONE: Oligonucleotide MPFL-4

(xi) SEQUENCE DESCRIPTION: SEQ ID NO:12:

CTAGTGATGT GACGCGGACG GACCT   25

(2) INFORMATION FOR SEQ ID NO:13:

    (i) SEQUENCE CHARACTERISTICS:
       (A) LENGTH: 11 amino acids
       (B) TYPE: amino acid
       (D) TOPOLOGY: linear

    (ii) MOLECULE TYPE: peptide

    (xi) SEQUENCE DESCRIPTION: SEQ ID NO:13:

Met Arg Met Ser Gln Val Arg Pro Arg His Ile
1               5                   10

(2) INFORMATION FOR SEQ ID NO:14:

    (i) SEQUENCE CHARACTERISTICS:
       (A) LENGTH: 57 amino acids
       (B) TYPE: amino acid
       (D) TOPOLOGY: linear

    (ii) MOLECULE TYPE: peptide

    (vii) IMMEDIATE SOURCE:
       (B) CLONE: Precursor polypeptide Met-Arg-Met-MPF-4

    (xi) SEQUENCE DESCRIPTION: SEQ ID NO:14:

Met Arg Met Ser Gln Val Arg Pro Arg His Ile Thr Ser Leu Glu Val
1               5                   10                  15

Ile Lys Ala Gly Pro His Cys Pro Thr Ala Gln Leu Ile Ala Thr Leu
                20                  25                  30

Lys Asn Gly Arg Lys Ile Cys Leu Asp Leu Gln Ala Pro Leu Tyr Lys
            35                  40                  45

Lys Ile Ile Lys Lys Leu Leu Glu Ser
    50                  55

(2) INFORMATION FOR SEQ ID NO:15:

    (i) SEQUENCE CHARACTERISTICS:
        (A) LENGTH: 58 base pairs
        (B) TYPE: nucleic acid
        (C) STRANDEDNESS: single
        (D) TOPOLOGY: linear

    (ii) MOLECULE TYPE: DNA (genomic)


   (vii) IMMEDIATE SOURCE:
        (B) CLONE: Oligonucleotide MPFL-5


    (xi) SEQUENCE DESCRIPTION: SEQ ID NO:15:

TATGCGTATG GAAGCTGAAG AAGATGGTGA CCTGCAGTGC CTGTGTGTGA AGACCACC   58


(2) INFORMATION FOR SEQ ID NO:16:

    (i) SEQUENCE CHARACTERISTICS:
        (A) LENGTH: 60 base pairs
        (B) TYPE: nucleic acid
        (C) STRANDEDNESS: single
        (D) TOPOLOGY: linear

    (ii) MOLECULE TYPE: DNA (genomic)


   (vii) IMMEDIATE SOURCE:
        (B) CLONE: Oligonucleotide MPFL-6


    (xi) SEQUENCE DESCRIPTION: SEQ ID NO:16:

GGGAGGTGGT CTTCACACAC AGGCACTGCA GGTCACCATC TTCTTCAGCT TCCATACGCA 60


(2) INFORMATION FOR SEQ ID NO:17:

    (i) SEQUENCE CHARACTERISTICS:
        (A) LENGTH: 27 amino acids
        (B) TYPE: amino acid
        (D) TOPOLOGY: linear

    (ii) MOLECULE TYPE: peptide

(xi) SEQUENCE DESCRIPTION: SEQ ID NO:17:

Met Arg Met Glu Ala Glu Glu Asp Gly Asp Leu Gln Cys Leu Cys Val
1               5                   10                  15

Lys Thr Thr Ser Gln Val Arg Pro Arg His Ile
            20                  25


(2) INFORMATION FOR SEQ ID NO:18:

    (i) SEQUENCE CHARACTERISTICS:
        (A) LENGTH: 73 amino acids
        (B) TYPE: amino acid
        (D) TOPOLOGY: linear

    (ii) MOLECULE TYPE: peptide


    (vii) IMMEDIATE SOURCE:
        (B) CLONE: Precursor polypeptide Met-Arg-Met-PF-4


    (xi) SEQUENCE DESCRIPTION: SEQ ID NO:18:

Met Arg Met Glu Ala Glu Glu Asp Gly Asp Leu Gln Cys Leu Cys Val
1               5                   10                  15

Lys Thr Thr Ser Gln Val Arg Pro Arg His Ile Thr Ser Leu Glu Val
            20                  25                  30

Ile Lys Ala Gly Pro His Cys Pro Thr Ala Gln Leu Ile Ala Thr Leu
        35                  40                  45

Lys Asn Gly Arg Lys Ile Cys Leu Asp Leu Gln Ala Pro Leu Tyr Lys
        50                  55                  60

Lys Ile Ile Lys Lys Leu Leu Glu Ser
65                  70


(2) INFORMATION FOR SEQ ID NO:19:

    (i) SEQUENCE CHARACTERISTICS:
        (A) LENGTH: 34 base pairs
        (B) TYPE: nucleic acid
        (C) STRANDEDNESS: single
        (D) TOPOLOGY: linear

    (ii) MOLECULE TYPE: DNA (genomic)

(xi) SEQUENCE DESCRIPTION: SEQ ID NO:19:

TCCCAGATGT CCCAGGTCCG TCCGCGTCAC ATCA    34

(2) INFORMATION FOR SEQ ID NO:20:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:20:

CTAGTGATGT GACGCGGACC TGGGACATCT    30

(2) INFORMATION FOR SEQ ID NO:21:

    (i) SEQUENCE CHARACTERISTICS:
        (A) LENGTH: 30 amino acids
        (B) TYPE: amino acid
        (D) TOPOLOGY: linear

    (ii) MOLECULE TYPE: peptide

    (xi) SEQUENCE DESCRIPTION: SEQ ID NO:21:

Met Arg Met Glu Ala Glu Glu Asp Gly Asp Leu Gln Cys Leu Cys Val
1               5                   10              15

Lys Thr Thr Ser Gln Met Ser Gln Val Arg Pro Arg His Ile
            20              25              30

(2) INFORMATION FOR SEQ ID NO:22:

    (i) SEQUENCE CHARACTERISTICS:
        (A) LENGTH: 76 amino acids
        (B) TYPE: amino acid
        (D) TOPOLOGY: linear

    (ii) MOLECULE TYPE: peptide

    (vii) IMMEDIATE SOURCE:
        (B) CLONE: Precursor polypeptide Met-Arg-Met-PF-4*

(xi) SEQUENCE DESCRIPTION: SEQ ID NO:22:

Met Arg Met Glu Ala Glu Glu Asp Gly Asp Leu Gln Cys Leu Cys Val
1               5               10                      15

Lys Thr Thr Ser Gln Met Ser Gln Val Arg Pro Arg His Ile Thr Ser
            20              25                  30

Leu Glu Val Ile Lys Ala Gly Pro His Cys Pro Thr Ala Gln Leu Ile
        35              40              45

Ala Thr Leu Lys Asn Gly Arg Lys Ile Cys Leu Asp Leu Gln Ala Pro
    50                  55              60

Leu Tyr Lys Lys Ile Ile Lys Lys Leu Leu Glu Ser
65                  70              75


(2) INFORMATION FOR SEQ ID NO:23:

        (i) SEQUENCE CHARACTERISTICS:
            (A) LENGTH: 22 amino acids
            (B) TYPE: amino acid
            (D) TOPOLOGY: linear

        (ii) MOLECULE TYPE: peptide


        (xi) SEQUENCE DESCRIPTION: SEQ ID NO:23:

Met Arg Met Glu Ala Glu Glu Asp Gly Asp Leu Gln Cys Leu Cys Val
1               5               10                      15

Lys Thr Thr Ser Gln Met
            20


(2) INFORMATION FOR SEQ ID NO:24:

        (i) SEQUENCE CHARACTERISTICS:
            (A) LENGTH: 58 base pairs
            (B) TYPE: nucleic acid
            (C) STRANDEDNESS: single
            (D) TOPOLOGY: linear

        (ii) MOLECULE TYPE: DNA (genomic)


        (vii) IMMEDIATE SOURCE:
            (B) CLONE: Oligonucleotide MPFL-11

(xi) SEQUENCE DESCRIPTION: SEQ ID NO:24:

TATGCGTATG GAAGCTGAAG AAGATGGTGA CCTGCAGTGC CTGTGTGTGA AGACCATG  58

(2) INFORMATION FOR SEQ ID NO:25:

    (i) SEQUENCE CHARACTERISTICS:
       (A) LENGTH: 60 base pairs
       (B) TYPE: nucleic acid
       (C) STRANDEDNESS: single
       (D) TOPOLOGY: linear

    (ii) MOLECULE TYPE: DNA (genomic)

    (vii) IMMEDIATE SOURCE:
       (B) CLONE: Oligonucleotide MPFL-12

    (xi) SEQUENCE DESCRIPTION: SEQ ID NO:25:

GGGACATGGT CTTCACACAC AGGCACTGCA GGTCACCATC TTCTTCAGCT TCCATACGCA 60

(2) INFORMATION FOR SEQ ID NO:26:

    (i) SEQUENCE CHARACTERISTICS:
       (A) LENGTH: 27 amino acids
       (B) TYPE: amino acid
       (D) TOPOLOGY: linear

    (ii) MOLECULE TYPE: peptide

    (ix) FEATURE:
       (A) NAME/KEY: CDS
       (B) LOCATION: 13..177

    (xi) SEQUENCE DESCRIPTION: SEQ ID NO:26:

Met Arg Met Glu Ala Glu Glu Asp Gly Asp Leu Gln Cys Leu Cys Val
1               5                       10                      15

Lys Thr Met Ser Gln Val Arg Pro Arg His Ile
                20                      25

(2) INFORMATION FOR SEQ ID NO:27:

    (i) SEQUENCE CHARACTERISTICS:
       (A) LENGTH: 73 amino acids
       (B) TYPE: amino acid
       (D) TOPOLOGY: linear

(ii) MOLECULE TYPE: peptide

(vii) IMMEDIATE SOURCE:
          (B) CLONE: Polypeptide Met-Arg-Met-PF-4+

      (xi) SEQUENCE DESCRIPTION: SEQ ID NO:27:

Met Arg Met Glu Ala Glu Glu Asp Gly Asp Leu Gln Cys Leu Cys Val
1               5                   10                  15

Lys Thr Met Ser Gln Val Arg Pro Arg His Ile Thr Ser Leu Glu Val
            20                  25                  30

le Lys Ala Gly Pro His Cys Pro Thr Ala Gln Leu Ile Ala Thr Leu
       35                  40                  45

Lys Asn Gly Arg Lys Ile Cys Leu Asp Leu Gln Ala Pro Leu Tyr Lys
       50                  55                  60

Lys Ile Ile Lys Lys Leu Leu Glu Ser
65                  70

(2) INFORMATION FOR SEQ ID NO:28:

      (i) SEQUENCE CHARACTERISTICS:
          (A) LENGTH: 19 amino acids
          (B) TYPE: amino acid
          (D) TOPOLOGY: linear

      (ii) MOLECULE TYPE: peptide

      (xi) SEQUENCE DESCRIPTION: SEQ ID NO:28:

Met Arg Met Glu Ala Glu Glu Asp Gly Asp Leu Gln Cys Leu Cys Val
1               5                   10                  15

Lys Thr Met

(2) INFORMATION FOR SEQ ID NO:29:

      (i) SEQUENCE CHARACTERISTICS:
          (A) LENGTH: 64 base pairs
          (B) TYPE: nucleic acid
          (C) STRANDEDNESS: single
          (D) TOPOLOGY: linear

      (ii) MOLECULE TYPE: DNA (genomic)

(xi) SEQUENCE DESCRIPTION: SEQ ID NO:29:

TATGCGTATG GAAGCTGAAG AAGATGGTGA CCTGCAGTGC CTGTGTGTGA AGACCACCTA 60

ATAG 64

(2) INFORMATION FOR SEQ ID NO:30:

      (i) SEQUENCE CHARACTERISTICS:
          (A) LENGTH: 66 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:30:

GATCCTATTA GGTGGTCTTC ACACACAGGC ACTGCAGGTC ACCATCTTCT TCAGCTTCCA 60

TACGCA 66

(2) INFORMATION FOR SEQ ID NO:31:

      (i) SEQUENCE CHARACTERISTICS:
          (A) LENGTH: 19 amino acids
          (B) TYPE: amino acid
          (D) TOPOLOGY: linear

    (ii) MOLECULE TYPE: peptide

    (vii) IMMEDIATE SOURCE:
          (B) CLONE: Polypeptide Met-Arg-Met-CPF-4A

    (xi) SEQUENCE DESCRIPTION: SEQ ID NO:31:

Met Arg Met Glu Ala Glu Glu Asp Gly Asp Leu Gln Cys Leu Cys Val
1              5                   10                 15

Lys Thr Thr

(2)  INFORMATION FOR SEQ ID NO:32:

    (i)  SEQUENCE CHARACTERISTICS:
        (A)  LENGTH: 51 base pairs
        (B)  TYPE: nucleic acid
        (C)  STRANDEDNESS: single
        (D)  TOPOLOGY: linear

   (ii)  MOLECULE TYPE: DNA (genomic)


   (ix)  FEATURE:
        (A)  NAME/KEY: CDS
        (B)  LOCATION: 18..44


   (xi)  SEQUENCE DESCRIPTION: SEQ ID NO:32:

TCTAGAGGGT ATTAATA ATG TAT ATT GAT TTT AAT AAG GAG GAA TAATCAT   51
                    Met Tyr Ile Asp Phe Asn Lys Glu Glu
                    1             5


(2)  INFORMATION FOR SEQ ID NO:33:

    (i)  SEQUENCE CHARACTERISTICS:
        (A)  LENGTH: 54 base pairs
        (B)  TYPE: nucleic acid
        (C)  STRANDEDNESS: single
        (D)  TOPOLOGY: linear

   (ii)  MOLECULE TYPE: DNA (genomic)


   (vii)  IMMEDIATE SOURCE:
        (B)  CLONE: Oligonucleotide MPF-1


   (xi)  SEQUENCE DESCRIPTION: SEQ ID NO:33:

AGCTTCGAAC ATATGTCCCA GGTCCGTCCG CGTCACATCA CTAGTCTGGA GGTG   54


(2)  INFORMATION FOR SEQ ID NO:34:

    (i)  SEQUENCE CHARACTERISTICS:
        (A)  LENGTH: 69 base pairs
        (B)  TYPE: nucleic acid
        (C)  STRANDEDNESS: single
        (D)  TOPOLOGY: linear

   (ii)  MOLECULE TYPE: DNA (genomic)

(vii) IMMEDIATE SOURCE:
    (B) CLONE: Oligonucleotide MPF-2

  (xi) SEQUENCE DESCRIPTION: SEQ ID NO:34:

ATCAAGGCCG GTCCGCACTG CCCGACTGCC CAACTGATTG CCACTCTGAA GAATGGTCGT 60

AAAATTTGC   69


(2) INFORMATION FOR SQ ID NO:35:

    (i) SEQUENCE CHARACTERISTICS:
      (A) LENGTH: 66 base pairs
      (B) TYPE: nucleic acid
      (C) STRANDEDNESS: single
      (D) TOPOLOGY: linear

    (ii) MOLECULE TYPE: DNA (genomic)


    (vii) IMMEDIATE SOURCE:
      (B) CLONE: Oligonucleotide MPF-3


    (xi) SEQUENCE DESCRIPTION: SEQ ID NO:35:

CTGGACCTGC AAGCCCCGCT GTACAAGAAA ATCATTAAGA AACTTCTGGA GTCTTAATAG 60

GATCCG   66


(2) INFORMATION FOR SEQ ID NO:36:

    (i) SEQUENCE CHARACTERISTICS:
      (A) LENGTH: 54 base pairs
      (B) TYPE: nucleic acid
      (C) STRANDEDNESS: single
      (D) TOPOLOGY: linear

    (ii) MOLECULE TYPE: DNA (genomic)


    (vii) IMMEDIATE SOURCE:
      (B) CLONE: Oligonucleotide MPF-4


    (xi) SEQUENCE DESCRIPTION: SEQ ID NO:36:

TGATCACCTC CAGACTAGTG ATGTGACGCG GACGGACCTG GGACATATGT TCGA   54

(2) INFORMATION FOR SEQ ID NO:37:

    (i) SEQUENCE CHARACTERISTICS:
        (A) LENGTH: 69 base pairs
        (B) TYPE: nucleic acid
        (C) STRANDEDNESS: single
        (D) TOPOLOGY: linear

    (ii) MOLECULE TYPE: DNA (genomic)


    (vii) IMMEDIATE SOURCE:
        (B) CLONE: Oligonucleotide MPF-5


    (xi) SEQUENCE DESCRIPTION: SEQ ID NO:37:

CCAGGCAAAT TTTACGACCA TTCTTCAGAG TGGCAATCAG TTGGGCAGTC GGGCAGTGCG 60

GACCGGCCT 69


(2) INFORMATION FOR SEQ ID NO:38:

    (i) SEQUENCE CHARACTERISTICS:
        (A) LENGTH: 66 base pairs
        (B) TYPE: nucleic acid
        (C) STRANDEDNESS: single
        (D) TOPOLOGY: linear

    (ii) MOLECULE TYPE: DNA (genomic)


    (vii) IMMEDIATE SOURCE:
        (B) CLONE: Oligonucleotide MPF-6


    (xi) SEQUENCE DESCRIPTION: SEQ ID NO:38:

AATTCGGATC CTATTAAGAC TCCAGAAGTT TCTTAATGAT TTTCTTGTAC AGCGGGGCTT 60

GCAGGT 66


(2) INFORMATION FOR SEQ ID NO:39:

    (i) SEQUENCE CHARACTERISTICS:
        (A) LENGTH: 35 base pairs
        (B) TYPE: nucleic acid
        (C) STRANDEDNESS: single
        (D) TOPOLOGY: linear

    (ii) MOLECULE TYPE: DNA (genomic)

(xi) SEQUENCE DESCRIPTION: SEQ ID NO:39:

TATGCGTATG TCCCAGGTCC GTCCGCGTCA CATCA    35


(2) INFORMATION FOR SEQ ID NO:40:

        (i) SEQUENCE CHARACTERISTICS:
            (A) LENGTH: 37 base pairs
            (B) TYPE: nucleic acid
            (C) STRANDEDNESS: single
            (D) TOPOLOGY: linear

    (ii) MOLECULE TYPE: DNA (genomic)

        (xi) SEQUENCE DESCRIPTION: SEQ ID NO:40:

CTAGTGATGT GACGCGGACG GACCTGGGAC ATACGCA    37


(2) INFORMATION FOR SEQ ID NO:41:

        (i) SEQUENCE CHARACTERISTICS:
            (A) LENGTH: 83 base pairs
            (B) TYPE: nucleic acid
            (C) STRANDEDNESS: single
            (D) TOPOLOGY: linear

    (ii) MOLECULE TYPE: DNA (genomic)

        (xi) SEQUENCE DESCRIPTION: SEQ ID NO:41:

TATGCGTATG GAAGCTGAAG AAGATGGTGA CCTGCAGTGC CTGTGTGTGA AGACCACCTC    60

CCAGGTCCGT CCGCGTCACA TCA    83


(2) INFORMATION FOR SEQ ID NO:42:

        (i) SEQUENCE CHARACTERISTICS:
            (A) LENGTH: 85 base pairs
            (B) TYPE: nucleic acid
            (C) STRANDEDNESS: single
            (D) TOPOLOGY: linear

    (ii) MOLECULE TYPE: DNA (genomic)

        (xi) SEQUENCE DESCRIPTION: SEQ ID NO:42:

CTAGTGATGT GACGCGGACG GACCTGGGAG GTGGTCTTCA CACACAGGCA CTGCAGGTCA    60

CCATCTTCTT CAGCTTCCAT ACGCA    85

2) INFORMATION FOR SEQ ID NO:43:

    (i) SEQUENCE CHARACTERISTICS:
        (A) LENGTH: 92 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:43:

TATGCGTATG GAAGCTGAAG AAGATGGTGA CCTGCAGTGC CTGTGTGTGA AGACCACCTC 60

CCAGATGTCC CAGGTCCGTC CGCGTCACAT CA   92

(2) INFORMATION FOR SEQ ID NO:44:

    (i) SEQUENCE CHARACTERISTICS:
        (A) LENGTH: 94 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:44:

CTAGTGATGT GACGCGGACG GACCTGGGAC ATCTGGGAGG TGGTCTTCAC ACACAGGCAC 60

TGCAGGTCAC CATCTTCTTC AGCTTCCATA CGCA   94

(2) INFORMATION FOR SEQ ID NO:45:

    (i) SEQUENCE CHARACTERISTICS:
        (A) LENGTH: 19 amino acids
        (B) TYPE: amino acid
        (D) TOPOLOGY: linear

    (ii) MOLECULE TYPE: peptide

    (xi) SEQUENCE DESCRIPTION: SEQ ID NO:45:

Met Arg Met Glu Ala Glu Glu Asp Gly Asp Leu Gln Cys Leu Cys Val
1               5                   10                  15
Lys Thr Met

```
(2) INFORMATION FOR SEQ ID NO:46:

        (i) SEQUENCE CHARACTERISTICS:
             (A) LENGTH: 58 base pairs
             (B) TYPE: nucleic acid
             (C) STRANDEDNESS: single
             (D) TOPOLOGY: linear

       (ii) MOLECULE TYPE: DNA (genomic)


      (xi) SEQUENCE DESCRIPTION: SEQ ID NO:46:

TATGCGTATG GAAGCTGAAG AAGATGGTGA CCTGCAGTGC CTGTGTGTGA AGACCATG   58


(2) INFORMATION FOR SEQ ID NO:47:

        (i) SEQUENCE CHARACTERISTICS:
             (A) LENGTH: 60 base pairs
            (B) TYPE: nucleic acid
             (C) STRANDEDNESS: single
             (D) TOPOLOGY: linear

       (ii) MOLECULE TYPE: DNA (genomic)

      (xi) SEQUENCE DESCRIPTION: SEQ ID NO:47:

GGGACATGGT CTTCACACAC AGGCACTGCA GGTCACCATC TTCTTCAGCT TCCATACGCA   60
```